(19) **Europäisches Patentamt**
**European Patent Office**
**Office européen des brevets**

(11) **EP 1 555 314 B1**

(12) **EUROPEAN PATENT SPECIFICATION**

(45) Date of publication and mention
of the grant of the patent:
**30.09.2009 Bulletin 2009/40**

(51) Int Cl.:
*C12N 9/54* (2006.01)     *C11D 3/386* (2006.01)

(21) Application number: **05075169.2**

(22) Date of filing: **26.06.1990**

(54) **Enzymes and detergent compositions**

Enzyme und Waschmittelzusammensetzungen

Enzymes et compositions de détergents

(84) Designated Contracting States:
**AT BE CH DE DK ES FR GB IT LI LU NL SE**

(30) Priority: **26.06.1989 DK 316989**

(43) Date of publication of application:
**20.07.2005 Bulletin 2005/29**

(62) Document number(s) of the earlier application(s) in
accordance with Art. 76 EPC:
**99102452.2 / 0 945 502**
**90910604.9 / 0 479 870**

(73) Proprietor: **Novozymes A/S**
**2880 Bagsvaerd (DK)**

(72) Inventors:
• **Branner, Sven**
**2800 Lyngby (DK)**
• **Hastrup, Sven**
**2200 Kobenhavn N (DK)**
• **Olsen, Ole Hvilsted**
**2700 Bronshoj (DK)**
• **Simonsen, Merete**
**3520 Farum (DK)**
• **Aaslyng, Dorrit**
**3500 Vaerløse (DK)**
• **Nørskov-Lauritsen, Leif**
**4733 Tappernøje (DK)**
• **Casteleijn, Eric**
**2907 CG Capelle a/d ljssel (NL)**
• **Egmond, Maarten Robert**
**3511 GW Utrecht (NL)**
• **Haverkamp, Johan**
**2661 KL Bergschenhoek (NL)**
• **Marugg, John David**
**3524 BL Utrecht (NL)**

• **Mooren, Arnoldus Theodorus Antonius**
**3136 TP Vlaardingen (NL)**

(74) Representative: **Stevens, Ian Edward**
**Potter Clarkson LLP**
**Park View House**
**58 The Ropewalk**
**Nottingham**
**NG1 5DD (GB)**

(56) References cited:
WO-A-87/05050       WO-A-88/08033
WO-A-89/06279       WO-A-89/09819
WO-A-89/09830       US-A- 4 760 025

• ALAN J. RUSSELL ET AL.: "Electrostatic effects
on modification of charged groups in the active
site cleft of subtilisin by protein engineering" J.
MOL. BIOL., vol. 193, 1987, pages 803-813,
XP002900491 London
• ALAN J. RUSSELL ET AL.: "Rational modification
of enzyme catalysis by engineering surface
charge" NATURE, vol. 328, 6 August 1987
(1987-08-06), pages 496-500, XP000887130
• WELLS J ET AL: "DESIGNING SUBSTRATE
SPECIFICITY BY PROTEIN ENGINEERING OF
ELECTROSTATIC INTERACTIONS"
PROCEEDINGS OF THE NATIONAL ACADEMY
OF SCIENCES OF USA, NATIONAL ACADEMY OF
SCIENCE. WASHINGTON, US, vol. 84, 1 March
1987 (1987-03-01), pages 1219-1223,
XP002008735 ISSN: 0027-8424
• WELLS J A ET AL: "SUBTILISIN- AN ENZYME
DESIGNED TO BE ENGINEERED" TRENDS IN
BIOCHEMICAL SCIENCES, ELSEVIER SCIENCE
PUBLISHERS BV., AMSTERDAM, NL, vol. 13,
August 1988 (1988-08), pages 291-297,
XP002027478 ISSN: 0376-5067

**(Cont. next page)**

Remarks:
The file contains technical information submitted after the application was filed and not included in this specification

Remarks:
The file contains technical information submitted after the application was filed and not included in this specification

**Description**

FIELD OF THE INVENTION

This invention relates to methods for preparing novel mutant enzymes or enzyme variants useful in formulating detergent compositions in exhibiting improved wash performance.

BACKGROUND OF THE INVENTION

In the detergent industry enzymes have for more than 20 years been implemented in washing formulations. Enzymes used in such formulations comprise proteases, lipases, amylases, cellulases, as well as other enzymes, or mixtures thereof. Commercially most important are proteases.

Although proteases have been used in the detergent industry for more than 20 years, it is still not exactly known which physical or chemical characteristics are responsible for a good washing performance or ability of a protease.

The currently used proteases have been found by isolating proteases from nature and testing them in detergent formulations.

BACILLUS PROTEASES

Enzymes cleaving the amide linkages in protein substrates are classified as proteases, or (interchangeably) peptidases (see Walsh, 1979, Enzymatic Reaction Mechanisms. W.H. Freeman and Company, San Francisco, Chapter 3). Bacteria of the Bacillus species secrete two extracellular species of protease, a neutral, or metalloprotease, and an alkaline protease which is functionally a serine endopeptidase, referred to as subtilisin. Secretion of these proteases has been linked to the bacterial growth cycle, with greatest expression of protease during the stationary phase, when sporulation also occurs. Joliffe et al. (1980, J. Bacterial 141:1199-1208) has suggested that Bacillus proteases function in cell wall turnover.

SUBTILISIN

A serine protease is an enzyme which catalyses the hydrolysis of peptide bonds, and in which there is an essential serine residue at the active site (White, Handler and Smith, 1973 "Principles of Biochemistry," Fifth Edition, McGraw-Hill Book Company, NY, pp. 271-272).

The bacterial serine proteases have molecular weights in the 20,000 to 45,000 range. They are inhibited by diisopropylfluorophosphate, but in contrast to metalloproteases, are resistant to ethylene diamino tetraacetic acid (EDTA) (although they are stabilized at high temperatures by calcium ions). They hydrolyse simple terminal esters and are similar in activity to eukaryotic chymotrypsin, also a serine protease. A more narrow term, alkaline protease, covering a sub-group, reflects the high pH optimum of some of the serine proteases, from pH 9.0 to 11.0 (for review, see Priest, 1977, Bacteriological Rev. 41:711-753).

In relation to the present invention a subtilisin is a serine protease produced by Gram-positive bacteria or fungi. A wide variety of subtilisins have been identified, and the amino acid sequence of a number of subtilisins have been determined. These include at least six subtilisins from Bacillus strains, namely, subtilisin 168, subtilisin BPN', subtilisin Carlsberg, subtilisin DY, subtilisin amylosacchariticus, and mesentericopeptidase (Kurihara et al., 1972, J.Biol.Chem. 247:5629-5631; Wells et al., 1983, Nucleic Acids Res. 11:7911-7925; Stahl and Ferrari, 1984, J.Bacteriol. 159:811-819, Jacobs et al., 1985, Nucl.Acids Res. 13:8913-8926; Nedkov et al., 1985, Biol.Chem. Hoppe-Seyler 366:421-430, Svendsen et al., 1986, FEBS Lett 196:228-232), one subtilisin from an actinomycetales, thermitase from Thermo-actinomyces vulgaris (Meloun et al., 1985, FEBS Lett. 1983: 195-200), and one fungal subtilisin, proteinase K from Tritirachium album (Jany and Mayer, 1985, Biol.Chem. Hoppe-Seyler 366:584-492).

Subtilisins are well-characterized physically and chemically. In addition to knowledge of the primary structure (amino acid sequence) of these enzymes, over 50 high resolution X-ray structures of subtilisin have been determined which delineate the binding of substrate, transition state, products, at least three different protease inhibitors, and define the structural consequences for natural variation (Kraut, 1977, Ann.Rev.Biochem. 46:331-358).

In the context of this invention, a subtilisin variant or mutated subtilisin protease means a subtilisin that has been produced by an organism which is expressing a mutant gene derived from a parent microorganism which possessed an original or parent gene and which produced a corresponding parent enzyme, the parent gene having been mutated in order to produce the mutant gene from which said mutated subtilisin protease is produced when expressed in a suitable host.

Random and site-directed mutations of the subtilisin gene have both arisen from knowledge of the physical and chemical properties of the enzyme and contributed information relating to subtilisin's catalytic activity, substrate specificity, tertiary structure, etc. (Wells et al., 1987, Proc.Natl.Acad.Sci. U.S.A. 84; 1219-1223; Wells et al., 1986, Phil.Trans.R.Soc.Lond.A. 317:415-423: Hwang and Warshel, 1987, Biochem. 26:2669-2673; Rao et al., 1987, Nature 328:551-554).

Especially site-directed mutagenesis of the subtilisin genes has attracted much attention, and various mutations are described in the following patent applications and patents:

EP publ. no. 130756 (GENENTECH) (corresponding to US Patent No. 4,760,025 (GENENCOR)) relating to site specific or randomly generated mutations in "carbonyl hydrolases" and subsequent screening of the mutated enzymes for various properties, such as $k_{cat}/F_m$ ratio, pH-activity profile, and oxidation stability. Apart from revealing that site-specific mutation is feasible, and that mutation of subtilisin BPN' in certain specified positions, i.e. $^{1}$Tyr, $^{32}$Asp, $^{155}$Asn, $^{104}$Tyr, $^{222}$Met, $^{166}$Gly, $^{64}$His, $^{169}$Gly, $^{189}$Phe, $^{33}$Ser, $^{221}$Ser, $^{217}$Tyr, $^{156}$Glu or $^{152}$Ala, provide for enzymes exhibiting altered properties, this application does not contribute to solving the problem of deciding where to introduce mutations in order to obtain enzymes with desired properties.

EP publ. no. 214435 (HENKEL) relating to cloning and expression of subtilisin Carlsberg and two mutants thereof. In this application no reason for mutation of $^{158}$Asp to $^{158}$Ser and $^{161}$Ser to $^{161}$Asp is provided.

In International patent publication No. WO 87/04461 (AMGEN) it is proposed to reduce the number of Asn-Gly sequences present in the parent enzyme in order to obtain mutated enzymes exhibiting improved pH and heat stabilities, in the application emphasis is put on removing, mutating, or modifying the $^{109}$Asn and the $^{218}$Asn residues in subtilisin BPN'.

International patent publication No. WO 87/05050 (GENEX) discloses random mutation and subsequent screening of a large number of mutants of subtilisin BPN' for improved properties. In the application mutations are described in positions $^{218}$Asn, $^{131}$Gly, $^{256}$Thr, $^{166}$Gly, $^{116}$Ala, $^{1886}$Ser, $^{126}$Leu, and $^{53}$Ser.

In EP application no. 87303761 (GENENTECH) it is described how homology considerations at both primary and tertiary structural levels may be applied to identify equivalent amino acid residues whether conserved or not. This information together with the inventors knowledge of the tertiary structure of subtilisin BPN' lead the inventors to select a number of positions susceptible to mutation with an expectation of obtaining mutants with altered properties. The positions so identified are: $^{124}$Met, $^{222}$Met, $^{204}$Tyr, $^{152}$Ala, $^{156}$Glu, $^{266}$Gly, $^{169}$Gly, $^{189}$Phe, $^{217}$Tyr. Also $^{155}$Asn, $^{21}$Tyr, $^{22}$Thr, $^{24}$Ser, $^{32}$Asp, $^{33}$Ser, $^{36}$Asp, $^{46}$Gly, $^{48}$Ala, $^{49}$Ser, $^{50}$Met, $^{77}$Asn, $^{87}$Ser, $^{94}$Lys, $^{95}$Val, $^{96}$Leu, $^{107}$Ile, $^{110}$Gly, $^{170}$Lys, $^{111}$Tyr, $^{172}$Pro, $^{197}$Asp, $^{199}$Met, $^{204}$Ser, $^{213}$Lys, and $^{221}$Ser, which positions are identified as being expected to influence various properties of the enzyme. Also, a number of mutations are exemplified to support these suggestions. In addition to single mutations in these positions the inventors also performed a number of multiple mutations. Further the inventors identify $^{225}$Gly, $^{67}$His, $^{126}$Leu, $^{135}$Leu, and amino acid residues within the segments 97-103, 126-129, 213-215, and 152-172 as having interest, but mutations in any of these positions are not exemplified.

EP publ. no. 260105 (GENENCOR) describes modification of certain properties in enzymes containing a catalytic triad by selecting an amino acid residue within about 15 A from the catalytic triad and replace the selected amino acid residue with another residue. Enzymes of the subtilisin type described in the present specification are specifically mentioned as belonging to the class of enzymes containing a catalytic triad. In subtilisins positions 222 and 217 are indicated as preferred positions for replacement.

International patent publication No. WO 88/06624 (GIST-BROCADES NV) discloses the DNA and amino acid sequences of a subtilisin protease designated PB92 almost 100% homologous in the amino acid sequence to the amino acid sequence of Subtilisin 309

International patent publication No. WO 88/07578 (GENENTECH) claims mutated enzymes derived from a precursor enzyme by replacement or modification of at least one catalytic group of an amino acid residue. The inventors state that by doing so a mutated enzyme is obtained that is reactive with substrates containing the modified or replaced catalytic group (substrate-assisted catalysis).

The general theory is based on <u>B. amyloliquefaciens</u> subtilisin (BPN'), where modifications have been described in positions $^{64}$His that was modified into $^{64}$Ala alone or in combination with a "helper" mutation of Ser-24-Cys. Modifications are also suggested in the amino acid residues $^{32}$Asp, and $^{221}$Ser, and a "helper" mutation of Ala-48-Glu.

International patent publication No. WO 88/08028 (GENEX) discloses genetic engineering around metal ion binding sites for the stabilization of proteins. This publication also uses Subtilisin BPN' as example and points at the following amino acid residues as candidates for substitution $^{172}$Pro (P172D, P172E), $^{131}$Gly (G131D), $^{76}$Asn (N76D; N76D+P172D(E)), $^{78}$Ser (S7BD). Further, suggestions are made for the combined mutants N76D+S78D+G131D+ P172D(E) ; N76D+G131D; S78D+G131D; S78D+P172D(E) AND S78D+G131D+P172D(E)

International patent publication No. WO 88/08033 (AMGEN) discloses a number of subtilisin analogues having a modified calcium binding site and either Asn or Gly replaced in any Asn-Gly sequence present in the molecule thereby obtaining enzymes exhibiting improved thermal and pH stability. One of the calcium binding sites is disclosed as involving the amino acid residues $^{41}$Asp, $^{75}$Leu, $^{76}$Asn, $^{77}$Asn, $^{78}$Ser, $^{791}$Ile, $^{80}$Gly, $^{81}$Val, $^{208}$Thr, and $^{214}$Tyr; other potential calcium binding sites are suggested at $^{140}$Asp, and $^{172}$Pro; $^{14}$ro, and $^{271}$Gln; and $^{172}$Pro and $^{195}$Glu or $^{197}$Asp. Also mutating the $^{109}$Asn and $^{218}$Asn positions is suggested. Mutants produced are N109S, N109S+N218S, N76D+N109S+N218S, N76D+N77D+N109S+N218S, N76D+179E+N109S+N218S.

International patent publication No. WO 88/08164 (GENEX) describes a method for identifying residues in a protein which may be substituted by a cysteine to permit formation of potentially stabilizing disulfide bonds. The method is based on detailed knowledge of the three dimensional structure of the protein and uses a computer for selecting the positions. In relation to subtilisin proteases, Subtilisin BPN' was used as a model system. Using the method on Subtilisin BPN' resulted in the suggestion of 11 candidates for introducing disulfide bonds (1:T22C+S87C, 2:V26C+L235C, 3: G47C+P57C, 4:M50C+N109C, 5:E156C+T164C, 6:V165C+K170C, 7:V165C+S191C, 8:Q206C+A216C, 9: A230C+V270C, 10:I234C+A274C, 11:H238C+W241C).
Of these 4 were produced (1, 2, 4, and 8) of which 2 did not provide any stabilizing effect (2 and 4). Further mutants were produced by combining two of these mutants with each other, and one with another mutation, viz. T22C+S87C+N218S, and T22C+S87C+Q206C+A216C. Also, a number of further unsuccessful mutants were produced, viz. A1C+S78C, S24C+S87C, K27C+S89C, A85C+A232C, I122C+V147C, S249C+A273C, and T253C+A273C.

Also, it has been shown by Thomas, Russell, and Fersht, Nature 318, 375-376(1985) that exchange of [99]Asp into [99]Ser in subtilisin BPN' changes the pH dependency of the enzyme.

In a subsequent article J. Mol. Biol. (1987)193, 803-813, the same authors also discuss the substitution of [156]Ser in place of [156]Glu.

Both these mutations are within a distance of about 15Å from the active [64]His.

In Nature 328, 496-500(1987) Russel and Fersht discuss the results of their experiments and present rules for changing pH-activity profiles by mutating an enzyme to obtain changes in surface charge.

ISOELECTRIC POINT (pI$_o$)

The isoelectric point, pI$_o$, is defined as the pH value where the enzyme molecule complex (with optionally attached metal or other ions) is neutral, i. e. the sum of electrostatic charges (net electrostatic charge, =NEC) on the complex is equal to zero. In this sum of course consideration of the positive or negative nature of the individual electrostatic charges must be taken into account.

The isoelectric point is conveniently calculated by using equilibrium considerations using pK values for the various charged residues in the enzyme in question and then by iteration find the pH value where the NEC of the enzyme molecule is equal to zero.

One problem with this calculation is that the pK values for the charged residues are dependent on their environment and consequently subject to variation. However, very good results are obtainable by allocating specific approximate pK values to the charged residues independently of the actual value. It is also possible to perform more sophisticated calculations, partly taking the environment into consideration.

The pI$_o$ may also be determined experimentally by isoelectric focusing or by titrating a solution containing the enzyme. Also, the various pK values for the charged residues may be determined experimentally by titration.

INDUSTRIAL APPLICATIONS OF SUBTILISINS

Proteases such as subtilisins have found much utility in industry, particularly in detergent formulations, as they are useful for removing proteinaceous stains.

At present the following proteases are known and many of them are marketed in large quantities in many countries of the world.

Subtilisin BPN' or Novo, available from e.g. SIGMA, St. Louis, U.S.A.

Subtilisin Carlsberg, marketed by Novo-Nordisk a/s (Denmark) as ALCALASE® and by IBIS (Holland) as MAXATASE®;

A Bacillus lentus subtilisin, marketed by NOVO INDUSTRI A/S (Denmark) as SAVINASE®;

Enzymes closely resembling SAVINASE® such as MAXACAL® marketed by IBIS, and OPTICLEAN® marketed by MILES KALI CHEMIE (FRG);

A Bacillus lentus subtilisin, marketed by Novo-Nordisk a/s (Denmark) as ESPERASE®;

KAZUSASE® marketed by SHOWA DENKO (Japan)

To be effective, however, such enzymes must not only exhibit activity under washing conditions, but must also be compatible with other detergent components during detergent production and storage.

For example, subtilisins may be used in combination with other enzymes active against other substrates, and the selected subtilisin should possess stability towards such enzymes, and also the selected subtilisin preferably should not catalyse degradation of the other enzymes. Also, the chosen subtilisin should be resistant to the action from other components in the detergent formulation, such as bleaching agents, oxidizing agents, etc., in particular an enzyme to be used in a detergent formulation should be stable with respect to the oxidizing power, calcium binding properties, and pH conditions rendered by the non-enzymatic components in the detergent during storage and in the wash liquor during wash.

The ability of an enzyme to catalyse the degradation of various naturally occurring substrates present on the objects to be cleaned during e.g. wash is often referred to as its washing ability, washability, detergency, or wash performance. Throughout this application the term wash performance will be used to encompass this property.

Naturally occurring subtilisins have been found to possess properties which are highly variable in relation to their washing power or ability under variations in parameters such as pH. Several of the above marketed detergent proteases, indeed, have a better performance than those marketed about 20 years ago, but for optimal performance each enzyme has its own specific conditions regarding formulation and wash conditions, e.g. pH, temperature, ionic strength (=I), active system (tensides, surfactants, bleaching agent, etc.), builders, etc.

As a consequence it is found that an enzyme possessing desirable properties at low pH and low I may be less attractive at more alkaline conditions and high I, or an enzyme exhibiting fine properties at high pH and high I may be less attractive at low pH, low I conditions.

The advent and development of recombinant DNA techniques has had a profound influence in the field of protein chemistry.

It has been envisaged that these techniques will make it possible to design peptides and proteins, such as enzymes, and hormones according to desired specifications, enabling the production of compounds exhibiting desired properties.

It is possible now to construct enzymes having desired amino acid sequences, and as indicated above a fair amount of research has been devoted to designing subtilisins with altered properties. Among the proposals the technique of producing and screening a large number of mutated enzymes as described in EP publ. no. 130756 (GENENTECH) (US Patent No. 4,760,025 (GENENCOR)) and International patent publ. no. WO 87/05050 (GENEX) correspond to the classical method of isolating native enzymes and screening them for their properties, but is more efficient through the knowledge of the presence of a large number of different mutant enzymes.

Since a subtilisin enzyme typically comprises 275 amino acid residues each capable of being 1 out of 20 possible naturally occurring amino acids, one very serious draw-back in that procedure is the very large number of mutations generated that has to be submitted to a preliminary screening prior to further testing of selected mutants showing interesting characteristics at the first screening, since no guidance is indicated in determining which amino acid residues to change in order to obtain a desired enzyme with improved properties for the use in question, such as, in this case formulating detergent compositions exhibiting improved wash performance under specified conditions of the wash liquor.

A procedure as outlined in these patent applications will consequently only be slightly better than the traditional random mutation procedures which have been known for years.

The other known techniques relate to changing specific properties, such as transesterification and hydrolysis rate (EP publ. no. 260105 (GENENCOR)), pH-activity profile (Thomas, Russell, and Fersht, supra), and substrate specificity (International patent publ. no. WO 88/07578 (GENENTECH)). None of these publications relates to changing the wash performance of enzymes.

A further technique that has evolved is using the detailed information on the three dimensional structure of proteins for analyzing the potential consequences of substituting certain amino acids. This approach has been used and is described in EP 260105 (GENENCOR), WO 88/07578 (GENENTECH), WO 88/08028 (GENEX), WO 88/08033 (AMGEN), and WO 88/08164 (GENEX)

Thus, as indicated above no relationship has yet been identified between well defined properties of an enzyme such as those mentioned above and the wash performance of an enzyme.

In unpublished International Patent Application no. PCT/DK 88/00002 (NOVO INDUSTRI A/S) it is proposed to use the concept of homology comparison to determine which amino acid positions should be selected for mutation and which amino acids should be substituted in these positions in order to obtain a desired change in wash performance.

By using such a procedure the task of screening is reduced drastically, since the number of mutants generated is much smaller, but with that procedure it is only foreseen that enzymes exhibiting the combined useful properties of the parent enzyme and the enzyme used in the comparison may be obtained.

The problem seems to be that although much research has been directed at revealing the mechanism of enzyme activity, still only little is known about the factors in structure and amino acid residue combination that determine the properties of enzymes in relation to their wash performance.

Consequently there still exists a need for further improvement and tailoring of enzymes to wash systems, as well as a better understanding of the mechanism of protease action in the practical use of cleaning or detergent compositions. Such an understanding could result in rules which may be applied for selecting mutations that with a reasonable degree of certainty will result in an enzyme exhibiting improved wash performance under specified conditions in a wash liquor.

## SUMMARY OF THE INVENTION

Further investigations into these problems have now surprisingly shown that one of the critical factors in the use of subtilisin enzymes in detergent compositions is the adsorption of the enzyme to the substrate, i.e. the material to be removed from textiles, hard surfaces or other materials to be cleaned.

Consequently, the present invention relates to mutations of the subtilisin 309 gene resulting in changed properties of the mutant subtilisin enzyme expressed by such a mutated gene, whereby said mutant subtilisin enzyme exhibit improved behaviour in detergent compositions. Mutations are generated at specific nucleic acids in the parent subtilisin gene responsible for the expression of specific amino acids in specific positions in the subtilisin enzyme.

According to the invention, there is provided a method of preparing a mutated subtilisin protease as set out in the attached claims.

The invention relates to mutations of the subtilisin 309 gene and ensuing mutant subtilisin 309 enzymes, which exhibit improved wash performance in different detergent compositions resulting in wash liquors of varying pH values.

## ABBREVIATIONS

## AMINO ACIDS

| A = | Ala = | Alanine |
|-----|-------|---------|
| V = | Val = | Valine |
| L = | Leu = | Leucine |
| I = | Ile = | Isoleucine |
| P = | Pro = | Proline |
| F = | Phe = | Phenylalanine |
| W = | Trp = | Tryptophan |
| M = | Met = | Methionine |
| G = | Gly = | Glycine |
| S = | Ser = | Serine |
| T = | Thr = | Threonine |
| C = | Cys = | Cysteine |
| Y = | Tyr = | Tyrosine |
| N = | Asn = | Asparagine |
| Q = | Gln = | Glutamine |
| D = | Asp = | Aspartic Acid |
| E = | Glu = | Glutamic Acid |
| K = | Lys = | Lysine |
| R = | Arg = | Arginine |
| H = | His = | Histidine |

## NUCLEIC ACID BASES

| A = | Adenine | |
|-----|---------|--|
| G = | Guanine | |
| C = | Cytosine | |
| T = | Thymine | (only in DNA) |
| U = | Uracil | (only in RNA) |

## MUTATIONS

In describing the various mutants produced or contemplated according to the invention, the following nomen-clatures were adapted for ease of reference:

Original amino acid(s) position(s) substituted amino acid(s)

According to this the substitution of Glutamic acid for glycine in position 195 is designated as:

Gly 195 Glu or G195E

a deletion of glycine in the same position is:

Gly 195 * or G195*

and insertion of an additional amino acid residue such as lysine is:

Gly 195 GlyLys or G195GK

Where a deletion is indicated in Table I, or present in a subtilisin not indicated in Table I, an insertion in such a position is indicated as:

* 36 Asp or *36D

for insertion of an aspartic acid in position 36
Multiple mutations are separated by pluses, i.e.:

Arg 170 Tyr + Gly 195 Glu or R170Y+G195E

representing mutations in positions 170 and 195 substituting tyrosine and glutamic acid for arginine and glycine, respectively.

TABLE I

COMPARISON OF AMINO ACID SEQUENCE FOR VARIOUS PROTEASES

| | |
|---|---|
| a = | subtilisin BPN' (Wells et al, 1983, supra) |
| b = | subtilisin amylosacchariticus (Kurihara et al, 1972, supra) |
| c = | subtilisin 168 (Stahl and Ferrari, 1984, supra) |
| d = | subtilisin mesentericopeptidase (Svendsen et al, 1986, supra) |
| e = | subtilisin DY (Nedkov et al, 1985, supra) |
| f = | subtilisin Carlsberg (Smith et al, 1968, supra) |
| g = | subtilisin Carlsberg (Jacobs et al, 1985, supra) |
| h = | subtilisin 309 (International Patent Application No. PCT/DK 88/00002) |
| i = | subtilisin 147 (International Patent Application No. PCT/DK 88/00002) |
| j = | thermitase (Meloun et al, 1985, supra) |
| k = | proteinase K (Betzel et al, 1988, Eur. J. Biochem. 178: 155 ff), and Gunkel et al, 1989, Eur. J. Biochem. 179: 185 ff) |
| l = | aqualysin (Kwon et al, 1988, Eur. J. Biochem. 173:491 ff) |
| m = | Bacillus PB92 protease (European Patent Publication No. 0 283 075) |
| n = | Protease TW7 (Tritirachium album)(International Patent Application No. PCT/US88/01040) |
| o = | Protease TW3 (Tritirachium album)(International Patent Application No. PCT/US88/01040) |
| *'= | assigned deletion |

```
No:              1                      10
a) *-*-*-*-*-*-*-A-Q-S-*-V-P-Y-G-V-S-Q-I-K-*-*-*-*-*-A-P-A-
b) *-*-*-*-*-*-*-A-Q-S-*-V-P-Y-G-I-S-Q-I-K-*-*-*-*-*-A-P-A-
c) *-*-*-*-*-*-*-A-Q-S-*-V-P-Y-G-I-S-Q-I-K-*-*-*-*-*-A-P-A-
d) *-*-*-*-*-*-*-A-Q-S-*-V-P-Y-G-I-S-Q-I-K-*-*-*-*-*-A-P-A-
e) *-*-*-*-*-*-*-A-Q-T-*-V-P-Y-G-I-P-L-I-K-*-*-*-*-*-A-D-K-
f) *-*-*-*-*-*-*-A-Q-T-*-V-P-Y-G-I-P-L-I-K-*-*-*-*-*-A-D-K-
g) *-*-*-*-*-*-*-A-Q-T-*-V-P-Y-G-I-P-L-I-K-*-*-*-*-*-A-D-K-
h) *-*-*-*-*-*-*-A-Q-S-*-V-P-W-G-I-S-R-V-Q-*-*-*-*-*-A-P-A-
i) *-*-*-*-*-*-*-*-Q-T-*-V-P-W-G-I-S-F-I-N-*-*-*-*-*-*-T-Q-Q-
j) Y-T-P-N-D-P-Y-F-S-S-*-R-Q-Y-G-P-Q-K-I-Q-*-*-*-*-*-A-P-Q-
k) *-*-*-*-*-*-*-A-A-Q-T-N-A-P-W-G-L-A-R-I-S-S-T-S-P-G-T-S-T-
l) *-*-*-*-*-*-*-A-T-Q-S-P-A-P-W-G-L-D-R-I-D-Q-R-D-L-P-L-S-N-
m) *-*-*-*-*-*-*-A-Q-S-*-V-P-W-G-I-S-R-V-Q-*-*-*-*-*-A-P-A-
n) *-*-*-*-*-*-*-A-T-Q-E-D-A-P-W-G-L-A-R-I-S-S-Q-E-P-G-G-T-T-
o) *-*-*-*-*-*-*-A-E-Q-R-N-A-P-W-G-L-A-R-I-S-S-T-S-P-G-T-S-T-


No:              20                     30                     40
a) L-H-S-Q-G-Y-T-G-S-N-V-K-V-A-V-I-D-S-G-I-D-S-S-H-P-D-L-*-
b) L-H-S-Q-G-Y-T-G-S-N-V-K-V-A-V-I-D-S-G-I-D-S-S-H-P-D-L-*-
c) L-H-S-Q-G-Y-T-G-S-N-V-K-V-A-V-I-D-S-G-I-D-S-S-H-P-D-L-*-
d) L-H-S-Q-G-Y-T-G-S-N-V-K-V-A-V-I-D-S-G-I-D-S-S-H-P-D-L-*-
e) V-Q-A-Q-G-Y-K-G-A-N-V-K-V-G-I-I-D-T-G-I-A-A-S-H-T-D-L-*-
f) V-Q-A-Q-G-F-K-G-A-N-V-K-V-A-V-L-D-T-G-I-Q-A-S-H-P-D-L-*-
g) V-Q-A-Q-G-F-K-G-A-N-V-K-V-A-V-L-D-T-G-I-Q-A-S-H-P-D-L-*-
h) A-H-N-R-G-L-T-G-S-G-V-K-V-A-V-L-D-T-G-I-*-S-T-H-P-D-L-*-
i) A-H-N-R-G-I-F-G-N-G-A-R-V-A-V-L-D-T-G-I-*-A-S-H-P-D-L-*-
j) A-W-*-D-I-A-E-G-S-G-A-K-I-A-I-V-D-T-G-V-Q-S-N-H-P-D-L-A-
k) Y-Y-Y-D-E-S-A-G-Q-G-S-C-V-Y-V-I-D-T-G-I-E-A-S-H-P-E-F-*-
l) S-Y-T-Y-T-A-T-G-R-G-V-N-V-Y-V-I-D-T-G-I-R-T-T-H-R-E-F-*-
m) A-H-N-R-G-L-T-G-S-G-V-K-V-A-V-L-D-T-G-I-*-S-T-H-P-D-L-*-
n) Y-T-Y-D-D-S-A-G-T-G-T-C-A-Y-I-I-D-T-G-I-Y-T-N-H-T-D-F-*-
o) Y-R-Y-D-D-S-A-G-Q-G-T-C-V-Y-V-I-D-T-G-V-E-A-S-H-P-E-F-*-
```

```
No:                    50                         60
a) *-K-V-A-G-G-A-S-M-V-P-S-E-T-N-P-F-*-*-Q-D-N-N-S-H-G-T-H-V-
b) *-N-V-R-G-G-A-S-F-V-P-S-E-T-N-P-Y-*-*-Q-D-G-S-S-H-G-T-H-V-
c) *-N-V-R-G-G-A-S-F-V-P-S-E-T-N-P-Y-*-*-Q-D-G-S-S-H-G-T-H-V-
d) *-N-V-R-G-G-A-S-F-V-P-S-E-T-N-P-Y-*-*-Q-D-G-S-S-H-G-T-H-V-
e) *-K-V-V-G-G-A-S-F-V-S-G-E-S-*-Y-N-*-*-T-D-G-N-G-H-G-T-H-V-
f) *-N-V-V-G-G-A-S-F-V-A-G-E-A-*-Y-N-*-*-T-D-G-N-G-H-G-T-H-V-
g) *-N-V-V-G-G-A-S-F-V-A-G-E-A-*-Y-N-*-*-T-D-G-N-G-H-G-T-H-V-
h) *-N-I-R-G-G-A-S-F-V-P-G-E-P-*-S-T-*-*-Q-D-G-N-G-H-G-T-H-V-
i) *-R-I-A-G-G-A-S-F-I-S-S-E-P-*-S-Y-*-*-H-D-N-N-G-H-G-T-H-V-
j) G-K-V-V-G-G-W-D-F-V-D-N-D-S-T-P-*-*-*-Q-N-G-N-G-H-G-T-H-C-
k) *-*-*-E-G-R-A-Q-M-V-K-T-Y-Y-Y-S-S-*-*-R-D-G-N-G-H-G-T-H-C-
l) *-*-*-G-G-R-A-R-V-G-Y-D-A-L-G-G-N-G-*-Q-D-C-N-G-H-G-T-H-V-
m) *-N-I-R-G-G-A-S-F-V-P-G-E-P-*-S-T-*-*-Q-D-G-N-G-H-G-T-H-V-
n) *-*-*-G-G-R-A-K-F-L-K-N-F-A-G-D-G-Q-D-T-D-G-N-G-H-G-T-H-V-
o) *-*-*-E-G-R-A-Q-M-V-K-T-Y-Y-A-S-S-*-*-R-D-G-N-G-H-G-T-H-C-


No:  70                     80                        90
a) A-G-T-V-A-A-L-*-N-N-S-I-G-V-L-G-V-A-P-S-A-S-L-Y-A-V-K-V-
b) A-G-T-I-A-A-L-*-N-N-S-I-G-V-L-G-V-A-P-S-A-S-L-Y-A-V-K-V-
c) A-G-T-I-A-A-L-*-N-N-S-I-G-V-L-G-V-S-P-S-A-S-L-Y-A-V-K-V-
d) A-G-T-I-A-A-L-*-N-N-S-I-G-V-L-G-V-A-P-S-A-S-L-Y-A-V-K-V-
e) A-G-T-V-A-A-L-*-D-N-T-T-G-V-L-G-V-A-P-N-V-S-L-Y-A-I-K-V-
f) A-G-T-V-A-A-L-*-D-N-T-T-G-V-L-G-V-A-P-S-V-S-L-Y-A-V-K-V-
g) A-G-T-V-A-A-L-*-D-N-T-T-G-V-L-G-V-A-P-S-V-S-L-Y-A-V-K-V-
h) A-G-T-I-A-A-L-*-N-N-S-I-G-V-L-G-V-A-P-S-A-E-L-Y-A-V-K-V-
i) A-G-T-I-A-A-L-*-N-N-S-I-G-V-L-G-V-A-P-S-A-D-L-Y-A-V-K-V-
j) A-G-I-A-A-A-V-T-N-N-S-T-G-I-A-G-T-A-P-K-A-S-I-L-A-V-R-V-
k) A-G-T-V-G-S-*-R-*-*-*-*-*-T-Y-G-V-A-K-K-T-Q-L-F-G-V-K-V-
l) A-G-T-I-G-G-V-*-*-*-*-*-*-T-Y-G-V-A-K-A-V-N-L-Y-A-V-R-V-
m) A-G-T-I-A-A-L-*-N-N-S-I-G-V-L-G-V-A-P-N-A-E-L-Y-A-V-K-V-
n) A-G-T-V-G-G-T-*-*-*-*-*-*-T-Y-G-V-A-K-K-T-S-L-F-A-V-K-V-
o) A-G-T-I-G-S-*-R-*-*-*-*-*-T-Y-G-V-A-K-K-T-Q-I-F-G-V-K-V-
```

```
No:        100                 110                      120
a) L-G-A-D-G-S-G-Q-Y-S-W-I-I-N-G-I-E-W-*-A-I-A-*-N-N-M-D-*-
b) L-D-S-T-G-S-G-Q-Y-S-W-I-I-N-G-I-E-W-*-A-I-A-*-N-N-M-D-*-
c) L-D-S-T-G-S-G-Q-Y-S-W-I-I-N-G-I-E-W-*-A-I-A-*-N-N-M-D-*-
d) L-D-S-T-G-S-G-Q-Y-S-W-I-I-N-G-I-E-W-*-A-I-A-*-N-N-M-D-*-
e) L-N-S-S-G-S-G-T-Y-S-A-I-V-S-G-I-E-W-*-A-T-Q-*-N-G-L-D-*-
f) L-N-S-S-G-S-G-S-Y-S-G-I-V-S-G-I-E-W-*-A-T-T-*-N-G-M-D-*-
g) L-N-S-S-G-S-G-T-Y-S-G-I-V-S-G-I-E-W-*-A-T-T-*-N-G-M-D-*-
h) L-G-A-S-G-S-G-S-V-S-S-I-A-Q-G-L-E-W-*-A-G-N-*-N-G-M-H-*-
i) L-D-R-N-G-S-G-S-L-A-S-V-A-Q-G-I-E-W-*-A-I-N-*-N-N-M-H-*-
j) L-D-N-S-G-S-G-T-W-T-A-V-A-N-G-I-T-Y-*-A-A-D-*-Q-G-A-K-*-
k) L-D-D-N-G-S-G-Q-Y-S-T-I-I-A-G-M-D-F-V-A-S-D-K-N-N-R-N-C-
l) L-D-C-N-G-S-G-S-T-S-G-V-I-A-G-V-D-W-V-*-T-*-R-N-H-R-R-P-
m) L-G-A-S-G-S-G-S-V-S-S-I-A-Q-G-L-E-W-*-A-G-N-*-N-G-M-H-*-
n) L-D-A-N-G-Q-G-S-N-S-G-V-I-A-G-M-D-F-V-T-K-D-A-S-S-Q-N-C-
o) L-N-D-Q-G-S-G-Q-Y-S-T-I-I-S-G-M-D-F-V-A-N-D-Y-R-N-R-N-C-


No:                          130                 140
a) *-*-*-*-V-I-N-M-S-L-G-G-P-S-G-S-A-A-L-K-A-A-V-D-K-A-V-A-
b) *-*-*-*-V-I-N-M-S-L-G-G-P-S-G-S-T-A-L-K-T-V-V-D-K-A-V-S-
c) *-*-*-*-V-I-N-M-S-L-G-G-P-T-G-S-T-A-L-K-T-V-V-D-K-A-V-S-
d) *-*-*-*-V-I-N-M-S-L-G-G-P-T-G-S-T-A-L-K-T-V-V-D-K-A-V-S-
e) *-*-*-*-V-I-N-M-S-L-G-G-P-S-G-S-T-A-L-K-Q-A-V-D-K-A-Y-A-
f) *-*-*-*-V-I-N-M-S-L-G-G-A-S-G-S-T-A-M-K-Q-A-V-D-N-A-Y-A-
g) *-*-*-*-V-I-N-M-S-L-G-G-P-S-G-S-T-A-M-K-Q-A-V-D-N-A-Y-A-
h) *-*-*-*-V-A-N-L-S-L-G-S-P-S-P-S-A-T-L-E-Q-A-V-N-S-A-T-S-
i) *-*-*-*-I-I-N-M-S-L-G-S-T-S-G-S-S-T-L-E-L-A-V-N-R-A-N-N-
j) *-*-*-*-V-I-S-L-S-L-G-G-T-V-G-N-S-G-L-Q-Q-A-V-N-Y-A-W-N-
k) P-K-G-V-V-A-S-L-S-L-G-G-G-Y-S-S-S-V-N-S-A-A-A-*-R-L-Q-S-
l) A-*-*-*-V-A-N-M-S-L-G-G-G-V-*-S-T-A-L-D-N-A-V-K-N-S-I-A-
m) *-*-*-*-V-A-N-L-S-L-G-S-P-S-P-S-A-T-L-E-Q-A-V-N-S-A-T-S-
n) P-K-G-V-V-V-N-M-S-L-G-G-P-S-S-S-A-V-N-R-A-A-A-*-E-I-T-S-
o) P-N-G-V-V-A-S-M-S-I-G-G-G-Y-S-S-S-V-N-S-A-A-A-*-N-L-Q-Q-
```

```
No:           150                 160                 170
a) S-G-V-V-V-V-A-A-A-G-N-E-G-T-S-G-S-S-S-T-V-G-Y-P-G-K-Y-P-
b) S-G-I-V-V-A-A-A-A-G-N-E-G-S-S-G-S-S-S-T-V-G-Y-P-A-K-Y-P-
c) S-G-I-V-V-A-A-A-A-G-N-E-G-S-S-G-S-T-S-T-V-G-Y-P-A-K-Y-P-
d) S-G-I-V-V-A-A-A-A-G-N-E-G-S-S-G-S-T-S-T-V-G-Y-P-A-K-Y-P-
e) S-G-I-V-V-V-A-A-G-N-S-G-S-S-G-S-Q-N-T-I-G-Y-P-A-K-Y-D-
f) R-G-V-V-V-V-A-A-A-G-N-S-G-N-S-G-S-T-N-T-I-G-Y-P-A-K-Y-D-
g) R-G-V-V-V-V-A-A-A-N-S-G-S-S-G-N-T-N-T-I-G-Y-P-A-K-Y-D-
h) R-G-V-L-V-V-A-A-S-G-N-S-G-A-*-G-S-I-S-*-*-*-Y-P-A-R-Y-A-
i) A-G-I-L-L-V-G-A-A-G-N-T-G-R-*-Q-G-V-N-*-*-*-Y-P-A-R-Y-S-
j) K-G-S-V-V-V-A-A-A-G-N-A-G-N-T-A-P-N-*-*-*-*-Y-P-A-Y-Y-S-
k) S-G-V-M-V-A-V-A-A-G-N-N-N-A-D-A-R-N-Y-S-*-*-*-P-A-S-E-P-
l) A-G-V-V-Y-A-V-A-A-G-N-D-N-A-N-A-C-N-Y-S-*-*-*-P-A-R-V-A-
m) R-G-V-L-V-V-A-A-S-G-N-S-G-A-*-G-S-I-S-*-*-*-Y-P-A-R-Y-A-
n) A-G-L-F-L-A-V-A-A-G-N-E-A-T-D-A-S-S-S-S-*-*-*-P-A-S-E-E-
o) S-G-V-M-V-A-V-A-A-G-N-N-N-A-D-A-R-N-Y-S-*-*-*-P-A-S-E-S-


No:           180                 190                 200
a) S-V-I-A-V-G-A-V-D-S-S-N-Q-R-A-S-F-S-S-V-G-P-E-L-D-V-M-A-
b) S-T-I-A-V-G-A-V-N-S-S-N-Q-R-A-S-F-S-S-A-G-S-E-L-D-V-M-A-
c) S-T-I-A-V-G-A-V-N-S-S-N-Q-R-A-S-F-S-S-A-G-S-E-L-D-V-M-A-
d) S-T-I-A-V-G-A-V-N-S-A-N-Q-R-A-S-F-S-S-A-G-S-E-L-D-V-M-A-
e) S-V-I-A-V-G-A-V-D-S-N-K-N-R-A-S-F-S-S-V-G-A-E-L-E-V-M-A-
f) S-V-I-A-V-G-A-V-D-S-N-S-N-R-A-S-F-S-S-V-G-A-E-L-E-V-M-A-
g) S-V-I-A-V-G-A-V-D-S-N-S-N-R-A-S-F-S-S-V-G-A-E-L-E-V-M-A-
h) N-A-M-A-V-G-A-T-D-Q-N-N-N-R-A-S-F-S-Q-Y-G-A-G-L-D-I-V-A-
i) G-V-M-A-V-A-A-V-D-Q-N-G-Q-R-A-S-F-S-T-Y-G-P-E-I-E-I-S-A-
j) N-A-I-A-V-A-S-T-D-Q-N-D-N-K-S-S-F-S-T-Y-G-S-V-V-D-V-A-A-
k) S-V-C-T-V-G-A-S-D-R-Y-D-R-R-S-S-F-S-N-Y-G-S-V-L-D-I-F-G-
l) E-A-L-T-V-G-A-T-T-S-S-D-A-R-A-S-F-S-N-Y-G-S-C-V-D-L-F-A-
m) N-A-M-A-V-G-A-T-D-Q-N-N-N-R-A-S-F-S-Q-Y-G-A-G-L-D-I-V-A-
n) S-A-C-T-V-G-A-T-D-K-T-D-T-L-A-E-Y-S-N-F-G-S-V-V-D-L-L-A-
o) S-I-C-T-V-G-A-T-D-R-Y-D-R-R-S-S-F-S-N-Y-G-S-V-L-D-I-F-A-
```

12

No:               210                          220

a) P-G-V-S-I-Q-S-T-L-P-G-N-*-K-*-Y-G-A-Y-N-G-T-S-M-A-S-P-H-

b) P-G-V-S-I-Q-S-T-L-P-G-G-*-T-*-Y-G-A-Y-N-G-T-S-M-A-T-P-H-

c) P-G-V-S-I-Q-S-T-L-P-G-G-*-T-*-Y-G-A-Y-N-G-T-S-M-A-T-P-H-

d) P-G-V-S-I-Q-S-T-L-P-G-G-*-T-*-Y-G-A-Y-N-G-T-S-M-A-T-P-H-

e) P-G-V-S-V-Y-S-T-Y-P-S-N-*-T-*-Y-T-S-L-N-G-T-S-M-A-S-P-H-

f) P-G-A-G-V-Y-S-T-Y-P-T-N-*-T-*-Y-A-T-L-N-G-T-S-M-A-S-P-H-

g) P-G-A-G-V-Y-S-T-Y-P-T-S-*-T-*-Y-A-T-L-N-G-T-S-M-A-S-P-H-

h) P-G-V-N-V-Q-S-T-Y-P-G-S-*-T-*-Y-A-S-L-N-G-T-S-M-A-T-P-H-

i) P-G-V-N-V-N-S-T-Y-T-G-N-*-R-*-Y-V-S-L-S-G-T-S-M-A-T-P-H-

j) P-G-S-W-I-Y-S-T-Y-P-T-S-*-T-*-Y-A-S-L-S-G-T-S-M-A-T-P-H-

k) P-G-T-S-I-L-S-T-W-I-G-G-*-S-*-T-R-S-I-S-G-T-S-M-A-T-P-H-

l) P-G-A-S-I-P-S-A-W-Y-T-S-D-T-A-T-Q-T-L-N-G-T-S-M-A-T-P-H-

m) P-G-V-N-V-Q-S-T-Y-P-G-S-*-T-*-Y-A-S-L-N-G-T-S-M-A-T-P-H-

n) P-G-T-D-I-K-S-T-W-N-D-G-R-T-K-I-I-S-*-*-G-T-S-M-A-S-P-H-

o) P-G-T-D-I-L-S-T-W-I-G-G-S-T-R-S-I-S-*-*-G-T-S-M-A-T-P-H-


No:               230                          240                          250

a) V-A-G-A-A-A-L-I-L-S-K-H-P-N-W-T-N-T-Q-V-R-S-S-L-E-N-T-T-

b) V-A-G-A-A-A-L-I-L-S-K-H-P-T-W-T-N-A-Q-V-R-D-R-L-E-S-T-A-

c) V-A-G-A-A-A-L-I-L-S-K-H-P-T-W-T-N-A-Q-V-R-D-R-L-E-S-T-A-

d) V-A-G-A-A-A-L-I-L-S-K-H-P-T-W-T-N-A-Q-V-R-D-R-L-E-S-T-A-

e) V-A-G-A-A-A-L-I-L-S-K-Y-P-T-L-S-A-S-Q-V-R-N-R-L-S-S-T-A-

f) V-A-G-A-A-A-L-I-L-S-K-H-P-N-L-S-A-S-Q-V-R-N-R-L-S-S-T-A-

g) V-A-G-A-A-A-L-I-L-S-K-H-P-N-L-S-A-S-Q-V-R-N-R-L-S-S-T-A-

h) V-A-G-A-A-A-L-V-K-Q-K-N-P-S-W-S-N-V-Q-I-R-N-H-L-K-N-T-A-

i) V-A-G-V-A-A-L-V-K-S-R-Y-P-S-Y-T-N-N-Q-I-R-Q-R-I-N-Q-T-A-

j) V-A-G-V-A-G-L-L-A-S-Q-G-R-S-*-*-A-S-N-I-R-A-A-I-E-N-T-A-

k) V-A-G-L-A-A-Y-L-M-T-L-G-K-T-T-A-A-S-A-C-R-*-Y-I-A-D-T-A-

l) V-A-G-V-A-A-L-Y-L-E-Q-N-P-S-A-T-P-A-S-V-A-S-A-I-L-N-G-A-

m) V-A-G-A-A-A-L-V-K-Q-K-N-P-S-W-S-N-V-Q-I-R-N-H-L-K-N-T-A-

n) V-A-G-L-G-A-Y-F-L-G-L-G-Q-K-V-Q-G-L-*-C-D-*-Y-M-V-E-K-G-

o) V-A-G-L-A-A-Y-L-M-T-L-G-R-A-T-A-S-N-A-C-R-*-Y-I-A-Q-T-A-

```
No:              260                      270        275
a) T-K-L-G-D-S-F-Y-Y-*-G-K-G-L-I-N-V-Q-A-A-A-Q
b) T-Y-L-G-D-S-F-Y-Y-*-G-K-G-L-I-N-V-Q-A-A-A-Q
c) T-Y-L-G-N-S-F-Y-Y-*-G-K-G-L-I-N-V-Q-A-A-A-Q
d) T-Y-L-G-S-S-F-Y-Y-*-G-K-G-L-I-N-V-Q-A-A-A-Q
e) T-N-L-G-D-S-F-Y-Y-*-G-K-G-L-I-N-V-E-A-A-A-Q
f) T-Y-L-G-S-S-F-Y-Y-*-G-K-G-L-I-N-V-E-A-A-A-Q
g) T-Y-L-G-S-S-F-Y-Y-*-G-K-G-L-I-N-V-E-A-A-A-Q
h) T-S-L-G-S-T-N-L-Y-*-G-S-G-L-V-N-A-E-A-A-T-R
i) T-Y-L-G-S-P-S-L-Y-*-G-N-G-L-V-H-A-G-R-A-T-Q
j) D-K-I-S-G-T-G-T-Y-W-A-K-G-R-V-N-A-Y-K-A-V-Q-Y
k) N-K-G-D-L-S-N-I-P-F-G-T-V-N-L-L-A-Y-N-N-Y-Q-A
l) T-T-G-R-L-S-G-I-G-S-G-S-P-N-R-L-L-Y-S-L-L-S-S-G-S-G
m) T-S-L-G-S-T-N-L-Y-*-G-S-G-L-V-N-A-E-A-A-T-R
n) L-K-D-V-I-Q-S-V-P-S-D-T-A-N-V-L-I-N-N-G-E-G-S-A
o) N-Q-G-D-L-S-N-I-S-F-G-T-V-N-L-A-Y-N-N-Y-Q-G
```

## DETAILED DESCAIPTION OF THE INVENTION

Above it was stated that the invention relates to mutated subtilisins in which the amino acid sequence has been changed through mutating the gene of the subtilisin 309 enzyme, which it is desired to modify, in codons responsible for the expression of amino acids located on or close to the surface of the resulting enzyme.

In the context of this invention a subtilisin is defined as a serine protease produced by gram-positive bacteria or fungi. In a more narrow sense, applicable to many embodiments of the invention, subtilisin also means a serine protease of gram-positive bacteria. According to another definition, a subtilisin is a serine protease, wherein the relative order of the amino acid residues in the catalytic triad is Asp - His - Ser (positions 32, 64, and 221). In a still more specific sense, many of the embodiments of the invention relate to serine proteases of gram-positive bacteria which can be brought into substantially unambiguous homology in their primary structure; with the subtilisins listed in Table I above.

Using the numbering system originating from the amino acid sequence of subtilisin BPN', which is shown in Table I above aligned with the amino acid sequence of a number of other known subtilisins, it is possible to indicate the position of an amino acid residue in a subtilisin enzyme unambiguously. Positions prior to amino acid residue number 1 in subtilisin BPN' are assigned a negative number, such as -6 for the N-terminal Y in thermitase, or 0, such as for the N-terminal A in proteinase K. Amino acid residues which are insertions in relation to subtilisin BPN' are numbered by the addition of letters in alphabetical order to the preceding subtilisin BPN' number, such as 12a, 12b, 12c, 12d, 12e for the "insert" S-T-S-P-G in proteinase K between [12]Ser and [13]Thr.

Using the above numbering system the positions of interest are:
1, 2, 3, 4, 6, 9, 10, 12, 14, 15, 17, 18, 19, 20, 21, 22, 24, 25, 27, 36, 37, 38, 40, 41, 43, 44, 45, 46, 49, 50, 51, 52, 53, 54, 55, 56, 57, 58, 59, 60, 61, 62, 75, 76, 77, 78, 79, 87, 89, 91, 94, 97, 98, 99, 100, 101, 103, 104, 105, 106, 107, 108, 109, 112, 113, 115, 116, 117, 118, 120, 126, 128, 129, 130, 131, 133, 134, 136, 137, 140, 141, 143, 144, 145, 146, 155, 156, 158, 159, 160, 161, 152, 163, 164, 165, 166, 107, 170, 171, 172, 173, 181, 182, 183, 184, 185, 186, 168, 189, 191, 192, 194, 195, 197, 204, 206, 209, 210, 211, 212, 213, 214, 215, 216, 217, 218, 235, 236, 237, 238, 239, 240, 241, 242, 243, 244, 245, 247, 248, 249, 251, 252, 253, 254, 255, 256, 257, 259, 260, 261, 262, 263, 265, 269, 271, 272, 275.

## ISOELECTRIC POINT ($pI_o$)

Assuming that the substrate under washing conditions has an electrostatic charge opposite to that of the enzyme, it might be expected that the adsorption and thus the wash performance of the enzyme to the substrate would be improved by increasing the net electrostatic charge, NEC, of the enzyme.

However, it was surprisingly found that a decrease in the NEC of the enzyme under such circumstances could result in an improved wash performance of the enzyme.

Stated differently, it was found that changing the isoelectric point, $pI_o$, of the enzyme in a direction to approach a lower pH, also shifted the pH of optimum wash performance of the enzyme to a lower value, meaning that in order to design an enzyme to a wash liquor of low pH, in which the enzyme is to be active, improvement in the wash performance of a know subtilisin enzyme may be obtained by mutating the gene for **the,** known subtilisin enzyme to obtain a mutant enzyme having a lower $pI_o$.

This finding led to experiments showing that the opposite also is feasible. Meaning that a known subtilisin enzyme may also be designed for use in high pH detergents by shifting its $pI_o$ to higher values, thereby shifting the wash performance pH optimum for the enzyme to higher pH values.

The present invention therefore in one aspect relates to a method of preparing a mutated subtilisin protease for use in a detergent composition, wherein the net electrostatic charge has been changed in comparison to the parent subtilisin 309 protease at the same pH, and in which proteases there are, relative to said parent protease, either fewer or more positively-charged amino acid residue(s) and/or more or fewer negatively-charged amino acid residue(s), or more or fewer positively-charged amino acid residue(s) and/or fewer or more negatively-charged amino acid residue(s) among the amino acid residues at position 245 by substitution,

and whereby said subtilisin protease has an isoelectric pH ($pI_o$) lower or higher, respectively, than that of said parent protease, [and the $pI_o$ of the mutated subtilisin protease is closer to the pH of the wash liquor formed by the detergent composition than is the $pI_o$ of the parent protease, such that the mutated subtilisin protease exhibits improved wash performance relative to the parent protease in said wash liquor.

In another preferred embodiment the invention relates to a method of preparing mutant subtilisin proteases for use in a detergent composition, wherein the NEC has been changed in comparison to the parent subtilisin 309 protease at the same pH, and in which proteases there are, relative to said parent protease, either sewer or more positively-charged amino acid residue(s) and/or more or fewer negatively-charged amino acid residue(s), or either more or fewer positively-charged amino acid residue(s) and/or fewer or more negatively-charged amino acid residue(s), among the amino acid residues at position 245 by substitution and at least one further mutation affecting an amino acid residue occupying a position chosen from the group of positions

1, 2, 3, 4, 6, 9, 10, 12, 14, 15, 17, 18, 19, 20, 21, 22, 24, 25, 36, 37, 38, 40, 41, 43, 44, 45, 46, 49, 50, 51, 52, 53, 54, 55, 56, 57, 58, 59, 60, 61, 62, 75, 76, 77, 78, 79, 87, 89, 91, 94, 97, 98, 99, 100, 101, 103, 104, 105, 106, 107, 108, 109, 112, 113, 115, 116, 117, 118, 120, 126, 128, 129, 130, 131, 133, 134, 136, 137, 140, 141, 143, 144, 145, 146, 155, 156, 158, 159, 160, 161, 162, 163, 164, 165, 166, 167, 170, 171, 172, 173, 181, 182, 183, 184, 185, 186, 188, 189, 191, 192, 194, 195, 197, 204, 206, 209, 210, 211, 212, 213, 214, 215, 216, 217, 218, 235, 236, 237, 238, 239, 240, 241, 242, 243, 244, 247, 248, 249, 251, 253, 254, 255, 256, 257, 259, 260, 261, 262, 263, 265, 269, 271, 272, 275,

and whereby said subtilisin protease has an isoelectric pH ($pI_o$) lower or higher, respectively, than that of said parent protease, and the $pI_o$ of the mutated subtilisin protease is closer to the pH of the wash liquor formed by the detergent composition than is the $pI_o$ of the parent protease, such that the mutated subtilisin protease exhibits improved wash performance relative to the parent protease in said wash liquor.

In these aspects the invention in short relates to mutant professes in which the $pI_o$ of the mutant protease is lower than the $pI_o$ of the parent subtilisin 209 protease, and in which the pH for optimum wash performance is also lower than the pH optimum for the parent protease; or mutant proteases wherein the $pI_o$ of the mutant protease is higher than the $pI_o$ of the parent protease, and in which the pH for optimum wash performance is also higher than the pH optimum for the parent protease.

It is generally believed (Thomas, Russell, and Fersht, supra) that kinetic properties can be influenced by changes in the electrostatic surface charge in the vicinity of the active site in the enzyme, but it has now surprisingly been found that changes in the kinetic properties of an enzyme can also be brought about by modifying surface charges remote from the active site.

Consequently the invention embraces mutant subtilisin enzymes, wherein one or more amino acid residues in a distance of more than 15Å from the catalytic triad of said enzyme has been changed in comparison to the amino acid sequence of its parent subtilisin 309 enzyme, and in a way to provide for a mutant protease having an isoelectric point (=$pI_o$) shifted in the same direction as it is desired to shift the pH for optimum wash performance of the enzyme, which pH optimum should be as close as possible to the pH of the wash liquor, wherein said mutant protease is intended for use.

The amino-acid sequence of the mutant protease may also contain an insertion mutation at position 36, for example to insert a negatively-charged amino-acid residue (e.g. D or E) or a neutral polar residue (such as A, Q, or N) or a positive amino-acid residue, such as R or K.

This is particularly applicable to subtilisin protease 309.

Insertion mutants at position 36 can have further mutations, for example at one or more of positions 120, 170, 195, 235, and/or 251, and/or at position 76.

Suitable mutations at position 76 are e.g. negatively charged residues such as N76D or N76E.

Mutations at position 36 (especially insertion of negative or polar neutral residue) and at position 76 (substitution by negatively-charged residue) can often have stabilising effect on the mutant protease, and can be used in combination. Mutations at for example one or more of positions 120, 170, 195, 235 and 251 have been found to be associated with increased enzyme activity. Even in cases where these latter mutations are associated individually with some loss of stability it can be acceptable and useful to combine them with mutations at one or both of positions 36 and 76.

Under some conditions it can be advantageous to arrange a further mutation in a mutant protease having an insertion of a negative amino-acid residue at position 36, to insert a positive charge elsewhere in the molecule. This can increase the water solubility of the resulting mutant protease, e.g. where the further mutation provides a positive charge, e.g. a positively-charged residue at position 213, e.g. T213K.

According to the invention the mutant subtilisin enzyme represents a mutation of a parent enzyme.

Mutated subtilisin proteases used in the examples, by way of reference, comprise one or more of the mutations:

| | |
|---|---|
| S001) | G195E |
| S002) | G195D |
| S003) | R170Y |
| S004) | R170Y+G195E |
| S005) | K251E |
| S006) | H120D |
| S008) | H120D+G195E |
| S009) | T71D |
| S010) | T71D+G195E |
| S011) | R170Y+K251E |
| S012) | R170Y+G195E+K251E |
| S013) | T71D+R170Y+K251E |
| S014) | T71D+R170Y+G195E+K251E |
| S015) | K235L |
| S016) | H120D+K235L |
| S017) | H120D+G195E+K235L |
| S234) | Q206D |
| S235) | *36D+N75D |
| S242) | *36Q+N76D+H120D+G195E+K235L |
| C001) | D14K |
| C002) | D120K |
| C003) | D140K |
| C004) | D14K+D1201K |
| C005) | K27D |
| C006) | K27D+D120K |
| C008) | D172K |
| C009) | D14K+D120K+D140K |
| C010) | D14K+D120K+D140K+D172K |
| C013) | N97D |
| C014) | S98D |
| C015) | T213D |
| C017) | S156E |
| C018) | N97D÷598D |
| C019) | N97D÷T213D |
| C022) | S98D÷T213D |
| C028) | N218D |
| C100) | V51D |
| C101) | E54T |
| C102) | E54Y |

The above observations about the $pI_o$ can be further utilized in a method for determining or selecting the position (s) and the amino acid(s) to be substituted for the amino acid(s) in a parent subtilisin 309 enzyme, whereby the selection is performed in a way whereby the calculated net electrostatic charge (=NEC) in a resulting mutant enzyme has been changed in comparison to the NEC in the parent enzyme of choice calculated at the same pH value.

Another way of expressing the principle covered by the invention is that the position(s) and the amino acid(s) to be substituted for the amino acid(s) in said parent subtilisin 309 enzyme is selected in a way whereby the total number of charges or total charge content (=TCC), and/or the NEC in a resulting mutant enzyme is changed in a way to provide for a mutant protease having an isoelectric point (=$pI_o$) shifted in the same direction as it is desired to shift the pH for optimum wash performance of the enzyme, which pH optimum should be as close as possible to the pH of the wash liquor, wherein said mutant protease is intended for use.

As indicated above the $pI_o$ of a macromolecule such as an enzyme is calculated as the pH where the NEC of the molecule is equal to zero. The procedure is exemplified in the examples below, but the principles are described in more detail here.

pK values are assigned to each potentially charged amino acid residue. Then the ratio of the occurrence of an amino acid residue at a given pH in charged or uncharged form (charged/uncharged, C/U(i)) is calculated for both negative and positive charge, by using the formulae Ia and Ib:

$$C/U(i) = \exp(\ln_{10}(pH - pK_i)) \qquad \text{(negative charge) (Ia)}$$

$$C/U(i) = \exp(\ln_{10}(pK_i - pH)) \qquad \text{(positive charge) (Ib)}$$

respectively.

From formulae Ia and Ib it is seen that at pH equal to $pK_i$, C/U(i) is equal to 1.

Then the relative charge, $Q_r(i)$, or charge contribution allocated to each charged residue is calculated by using the formulae IIa and IIb:

$$Q_r(i) = C/U(i)/(1 + C/U(i)) \qquad \text{(negative charge) (IIa)}$$

$$Q_r(i) = -C/U(i)/(1 + C/U(i)) \qquad \text{(positive charge) (IIb)}$$

The pH value where the sum of all the charge contributions from the charged residues is equal to zero is found by iteration or through interpolation in a sufficiently dense pH-charge sum table.

DETERGENT COMPOSITIONS COMPRISING THE MUTANT ENZYMES

The present invention also discloses the use of the mutant enzymes of the invention in cleaning and detergent compositions.

Such compositions comprise in addition to any one or more of the mutant subtilisin enzymes in accordance to any of the preceding aspects of the invention alone or in combination any of the usual components included in such compositions which are well-known to the person skilled in the art.

Such components comprise builders, such as phosphate or zeolite builders, surfactants, such anionic, cationic or non-ionic surfactants, polymers, such as acrylic or equivalent polymers, bleach systems, such as perborate- or amino-containing bleach precursors or activators, structurants, such as silicate structurants, alkali or acid to adjust pH, humectants, and or neutral inorganic salts.

In several useful embodiments the detergent compositions can be formulated as follows:

a) A detergent composition formulated as a detergent powder containing phosphate builder, anionic surfactant, nonionic surfactant, acrylic or equivalent polymer, perborate bleach precursor, amino-containing bleach activator, silicate or other structurant, alkali to adjust to desired pH in use, and neutral inorganic salt.

b) A detergent composition formulated as a detergent powder containing zeolite builder, anionic surfactant, nonionic surfactant, acrylic or equivalent polymer, perborate bleach precursor, amino-containing bleach activator, silicate or other structurant, alkali to adjust to desired pH in use, and neutral inorganic salt.

c) A detergent composition formulated as an aqueous detergent liquid comprising anionic surfactant, nonionic surfactant, humectant, organic acid, caustic alkali, with a pH adjusted to a value between 9 and 10.

d) A detergent composition formulated as a nonaqueous detergent liquid comprising a liquid nonionic surfactant consisting essentially of linear alkoxylated primary alcohol, triacetin, sodium triphosphate, caustic alkali, perborate monohydrate bleach precursor, and tertiary amine bleach activator, with a pH adjusted to a value between about 9 and 10.

e) A detergent composition formulated as a detergent powder in the form of a granulate having a bulk density of at least 550 g/l, e.g. at least 600 g/l, containing anionic and nonionic surfactants, e.g. anionic surfactant and a mixture of nonionic surfactants with respective alkoxylation degrees about 7 and about 3, low or substantially zero neutral inorganic salt, phosphate builder, perborate bleach precursor, tertiary amine bleach activator, sodium silicate, and minors and moisture.

f) A detergent composition formulated as a detergent powder in the form of a granulate having a bulk density of at least 600 g/l, containing anionic surfactant and a mixture of nonionic surfactants with respective alkoxylation degrees about 7 and about 3, low or substantially zero neutral inorganic salt, zeolite builder, perborate bleach precursor, tertiary amine bleach activator, sodium silicate, and minors and moisture.

g) A detergent composition formulated as a detergent powder containing anionic surfactant, nonionic surfactant, acrylic polymer, fatty acid soap, sodium carbonate, sodium sulphate, clay particles with or without amines, perborate bleach precursor, tertiary amine bleach activator, sodium silicate, and minors and moisture.

h) A detergent composition formulated as a detergent (soap) bar containing soap based on pan-saponified mixture of tallow and coconut oil, neutralised with orthophosphoric acid, mixed with protease, also mixed with sodium formate, borax, propylene glycol and sodium sulphate, and then plodded on a soap production line.

j) An enzymatic detergent composition formulated to give a wash liquor pH of 9 or less when used at a rate co=responding to 0.4-0.8 g/l surfactant.

k) An enzymatic detergent composition formulated to give a wash liquor pH of 8.5 or more when used at a rate corresponding to 0.4-0.8 g/l surfactant.

l) An enzymatic detergent composition formulated to give a wash liquor ionic strength of 0.03 or less, e.g. 0.02 or less, when used at a rate corresponding to 0.4-0.8 g/l surfactant.

m) An enzymatic detergent composition formulated to give a wash liquor ionic strength of 0.01 or more, e.g. 0.02 or more, when used at a rate corresponding to 0.4-0.8 g/l surfactant.

DETERGENT COMPOSITIONS COMPRISING MUTANT ENZYMES IN COMBINATION WITH LIPASE

It has surprisingly been found that a decrease in the isoelectric point, pI, and hence net charge of a subtilisin type protease under washing conditions, can result in not only an improved wash performance of the enzyme but also an improved compatibility with lipase.

It has also been surprisingly found that compatibility of protease with lipase is influenced not only by the pI but also by the positions at which the charges are located relative to the active site of the protease: The introduction of negative charge or removal of positive charge closer to the active site gives stronger improvement of compatibility of protease with lipase.

Accordingly, the invention discloses enzymatic detergent compositions, comprising lipase and also comprising mutated subtilisin protease produced according to the invention, wherein the net molecular electrostatic charge of the mutated protease has been changed by insertion, deletion or substitution of amino-acid residues in comparison to the parent protease, and wherein, in said protease, there are, relative to said parent subtilisin 309 protease, fewer positively-charged amino-acid residue (s) and/or more negatively-charged amino-acid residue(s), whereby said subtilisin protease has an isoelectric pH ($pI_o$) lower than that of said parent protease.

One preferred class of lipases for such use originates in Gram-negative bacteria, and includes e.g. lipase enzymes of the groups defined in EP 0 205 208 and 0 206 390 ( both to Unilever), (hereby incorporated by reference), including lipases immunologically related to those from certain Ps. fluorescens, P. gladioli and Chromobacter strains.

Preferred embodiments of mutant subtilisin protease enzyme for use in conjunction, with lipase as aforesaid possess one or more mutations at the site of an amino-acid residue located within the range of about 15A-20A from the active site, especially for example at positions 170, 120, or 195.

The lipase can usefully be added in the form of a granular composition, (alternatively a solution or a slurry), of lipolytic enzyme with carrier material (e.g. as in EP 258068 (Novo Nordisk A/S) and' Savinase® and Lipolase® products of Novo Nordisk A/S).

The added amount of lipase can be chosen within wide limits, for example 50 to 30,000 LU/g per gram of the surfactant system or of the detergent composition, e.g. often at least 100 LU/g, very usefully at least 500 LU/g, sometimes preferably above 1000, above 2000 LU/g or above 4000 LU/g or more, thus very often within the range 50-4000 LU/g and possibly within the range 200-1000 LU/g. In this specification lipase units are defined as they are in EP 258068.

The lipolytic enzyme can be chosen from among a wide range of lipases: in particular the lipases described in for example the following patent specifications, EP 214761 (Novo Nordisk A/S), EP 0 258 068 and especially lipases showing immunological cross-reactivity with antisera raised against lipase from Thermomyces lanuginosus ATCC 22070, EP 0 205 208 and EP 0 206 390 and especially lipases showing immunological cross-reactivity with antisera raised against lipase from Chromobacter viscosum var lipolyticum NRRL B-3673, or against lipase from Alcaligenes PL-679, ATCC 31371 and FERM-P 3783, also the lipases described in specifications WO 87/00859 (Gist-Brocades) and EP 0 204 284 (Sapporo Breweries). Suitable in particular are for example the following commercially available lipase preparations: Novo Lipolase®, Amano lipases CE, P, B, AP, M-AP, AML, and CES, and Meito lipases MY-30, OF, and PL, also Esterase® MM, Lipozyr®, SP225, SP285, Saiken lipase, Enzeco lipase, Toyo Jozo lipase and Diosynth lipase (Trade Marks).

Genetic engineering of the enzymes can be achieved by extraction of an appropriate lipase gene, e.g. the gene for lipase from Thermomyces lanuginosus or from a mutant thereof, and introduction and expression of the gene or derivative thereof in a suitable producer organism such as an Aspergillus. The techniques described in WO 88/02775 (Novo Nordisk A/S), EP 0 243 338 (Labofina), EP 0 268 452 (Genencor) and notably EP 0 305 216 (Novo Nordisk A/S) or EP 0 283 075 (Gist-Brocades) may be applied and adapted.

Similar considerations apply mutatis mutandis in the case of other enzymes, which may also be present. Without limitation: Amylase can for example be used when present in an amount in the range about 1 to about 100 MU (maltose units) per gram of detergent composition, (or 0.014-1.4, e.g. 0.07-0.7, KNU/g (Novo units)). Cellulase can for example be used when present in an amount in the range about 0.3 to about 35 CEVU units per gram of the detergent composition.

The detergent compositions may furthermore include the following usual detergent ingredients in the usual amounts. They may be built or unbuilt, and may be of the zero-P type (i.e. not containing any phosphorus-containing builders). Thus the composition may contain in aggregate for example from 1-50%, e.g. at least about 5% and often up to about 35-40% by weight, of one or more organic and/or inorganic builders. Typical examples of such builders include those already mentioned above, and more broadly include alkali metal ortho, pyro, and tripolyphosphates, alkali metal carbonates, either alone or in admixture with calcite, alkali metal citrates, alkali metal nitrilotriacetates, carboxymethyloxysuccinates, zeolites, polyacetalcarboxylates and so on.

Furthermore, the detergent compositions may contain from 1-35% of a bleaching agent or a bleach precursor or a system comprising bleaching agent and/or precursor with activator therefor. Further optional ingredients are lather boosters, foam depressors, anti-corrosion agents, soil-suspending agents, sequestering agents, anti-soil redeposition agents, perfumes, dyes, stabilising agents for the enzymes and so on.

The compositions can be used for the washing of textile materials, especially but without limitation cotton and polyester-based textiles and mixtures thereof. Especially suitable are for example washing processes carried out at temperatures of about 60-65 deg C or lower, e.g. about 30°C-35°C or lower. It can be very suitable to use the compositions at a rate sufficient to provide about e.g. 0.4-0.8 g/l surfactant in the wash liquor, although it is of course possible to use lesser or greater concentrations if desired. Without limitation it can for example be stated that a use-rate from about 3 g/l and up to about 6 g/l of the detergent formulation is suitable for use in the case when the formulations are as in the Examples.

METHOD FOR PRODUCING MUTATIONS IN SUBTILISIN GENES

Many methods for introducing mutations into genes are well known in the art. After a brief discussion of cloning subtilisin genes, methods for generating mutations in both random sites, and specific sites, within the subtilisin gene will be discussed.

CLONING A SUBTILISIN GENE

The gene encoding subtilisin may be cloned from any Gram-positive bacteria or fungus by various methods, well known in the art. First a genomic, and/or cDNA library of DNA must be constructed using chromosomal DNA or messenger RNA from the organism that produces the subtilisin to be studied. Then, if the amino-acid sequence of the subtilisin is known, homologous, labelled oligonucleotide probes may be synthesized and used to identify subtilisin-encoding clones from a genomic library of bacterial DNA, or from a fungal cDNA library. Alternatively, a labelled oligonucleotide probe containing sequences homologous to subtilisin from another strain of bacteria or fungus could be used as a probe to identify subtilisin-encoding clones, using hybridization and washing conditions of lower stringency.

Yet another method for identifying subtilisin-producing clones would involve inserting fragments of genomic DNA into an expression vector, such as a plasmid, transforming protease-negative bacteria with the resulting genomic DNA library, and then plating the transformed bacteria onto agar containing a substrate for subtilisin, such as skim milk. Those bacteria containing subtilisin-bearing plasmid will produce colonies surrounded by a halo of clear agar, due to digestion of the skim milk by excreted subtilisin.

GENERATION OF RANDOM MUTATIONS IN THE SUBTILISIN GENE

Once the subtilisin gene has been cloned into a suitable vector, such as a plasmid, several methods can be used to introduce random mutations into the gene.

One method would be to incorporate the cloned subtilisin gene, as part of a retrievable vector, into a mutator strain of Eschericia coli.

Another method would involve generating a single stranded form of the subtilisin gene, and then annealing the fragment of DNA containing the subtilisin gene with another DNA fragment such that a portion of the subtilisin gene remained single stranded. This discrete, single stranded region could then be exposed to any of a number of mutagenizing agents, including, but not limited to, sodium bisulfite, hydroxylamine, nitrous acid, formic acid, or hydralazine. A specific example of this method for generating random mutations is described by Shortle and Nathans (1978, Proc. Natl. Acad. Sci. U.S.A., 75: 2170-2174). According to the shortle and Nathans method, the plasmid bearing the subtilisin gene would be nicked by a restriction enzyme that cleaves within the gene. This nick would be widened into a gap using the exonuclease action of DNA polymerase I. The resulting single-stranded gap could then be mutagenized using any one of the above mentioned mutagenizing agents.

Alternatively, the subtilisin gene from a Bacillus species including the natural promoter and other control sequences could be cloned into a plasmid vector containing replicons for both E. coli and B. subtilis, a selectable phenotypic marker and the M13 origin of replication for production of single-stranded plasmid DNA upon superinfection with helper phage IR1. Single-stranded plasmid DNA containing the cloned subtilisin gene is isolated and annealed with a DNA fragment containing vector sequences but not the coding region of subtilisin, resulting in a gapped duplex molecule. Mutations are introduced into the subtilisin gene either with sodium bisulfite, nitrous acid or formic acid or by replication in a mutator strain of E. coli as described above. Since sodium bisulfite reacts exclusively with cytosine in a single-stranded DNA, the mutations created with this mutagen are restricted only to the coding regions. Reaction time and bisulfite concentration are varied in different experiments such that from one to five mutations are created per subtilisin gene on average. Incubation of 10 mg of gapped duplex DNA in 4 M Na-bisulfite, pH. 6.0, for 9 minutes at 37°C in a reaction volume of 400 ml, deaminates about 1% of cytosines in the single-stranded region. The coding region of mature subtilisin contains about 200 cytosines, depending on the DNA strand. Advantageously, the reaction time is varied from about 4 minutes (to produce a mutation frequency of about one in 200) to about 20 minutes (about 5 in 200).

After mutagenesis the gapped molecules are treated in vitro with DNA polymerase I (Klenow fragment) to make fully double-stranded molecules and fix the mutations. Competent E. coli are then transformed with the mutagenized DNA to produce an amplified library of mutant subtilisins. Amplified mutant libraries can also be made by growing the plasmid DNA in a Mut D strain of. E. coli which increases the range for mutations due to its error prone DNA polymerase.

The mutagens nitrous acid and formic acid may also be used to produce mutant libraries. Because these chemicals are not as specific for single-stranded DNA as sodium bisulfite, the mutagenesis reactions are performed according to the following procedure. The coding portion of the subtilisin gene is cloned in M13 phage by standard methods and single stranded phage DNA prepared. The single-stranded DNA is then reacted with 1 M nitrous acid pH. 4.3 for 15-60 minutes at 23°C or 2.4 M formic acid for 1-5 minutes at 23°C. These ranges of reaction times produce a mutation frequency of from 1 in 1000 to 5 in 1000. After mutagenesis, a universal primer is annealed to the M13 DNA and duplex DNA is synthesized using the mutagenized single-stranded DNA as a template so that the coding portion of the subtilisin gene becomes fully double-stranded. At this point the coding region can be cut out of the M13 vector with restriction enzymes and ligated into an unmutagenized expression vector so that mutations occur only in the restriction fragment. (Myers et al., Science 229:242-257 (1985)).

GENERATION OF SITE DIRECTED MUTATIONS IN THE SUBTILISIN GENE

Once the subtilisin gene has been cloned, and desirable sites for mutation identified, these mutations can be introduced using synthetic oligonucleotides. These oligonucleotides contain nucleotide sequences flanking the desired mutation sites; mutant nucleotides are inserted during oligonucleotide synthesis. In a preferred method, a single stranded gap of DNA, bridging the subtilisin gene, is created in a vector bearing the subtilisin gene. Then the synthetic nucleotide, bearing the desired mutation, is annealed to a homologous portion of the single-stranded DNA. The remaining gap is then filled in by DNA polymerase I (Klenow fragment) and the construct is ligated using T4 ligase. A specific example of this method is described in Morinaga et al., (1984, Biotechnology 2:646-639). According to Morinaga et al., a fragment within the gene is removed using restriction endonuclease. The vector/gene, now containing a gap, is then denatured and hybridized to a vector/gene which, instead of containing a gap, has been cleaved with another restriction endonuclease at a site outside the area involved in the gap. A single-stranded region of the gene is then available for hybridization with mutated oligonucleotides, the remaining gap is filled in by the Klenow fragment of DNA polymerase I, the insertions are ligated with T4 DNA ligase, and, after one cycle of replication, a double-stranded plasmid bearing the desired mutation is produced. The Morinaga method obviates the additional manipulation of constructing new restriction sites, and therefore facilitates the generation of mutations at multiple sites. U.S. Patent number 4,760,025, by Estell et al., issued July 26, 1988, is able to introduce oligonucleotides bearing multiple mutations by performing minor alterations of the cassette, however, an even greater variety of mutations can be introduced at any one time by the Morinaga method, because a multitude of oligonucleotides, of various lengths, can be introduced.

EXPRESSION OF SUBTILISIN MUTANTS

According to the invention, a mutated subtilisin gene produced by methods described above, or any alternative methods known in the art, can be expressed, in enzyme form, using an expression vector. An expression vector generally falls under the definition of a cloning vector, since an expression vector usually includes the components of a typical cloning vector, namely, an element that permits autonomous replication of the vector in a microorganism independent of the genome of the microorganism, and one or more phenotypic markers for selection purposes. An expression vector includes control sequences encoding a promoter, operator, ribosome binding site, translation initiation signal, and, optionally, a repressor gene or various activator genes. To permit the secretion of the expressed protein, nucleotides encoding a "signal sequence" may be inserted prior to the coding sequence of the gene. For expression under the direction of control sequences, a target gene to be treated according to the invention is operably linked to the control sequences in the proper reading frame. Promoter sequences that can be incorporated into plasmid vectors, and which can support the transcription of the mutant subtilisin gene, include but are not limited to the prokaryotic β-lactamase promoter (Villa-Kamaroff, et al., 1978, Proc. Natl. Acad. Sci. U.S.A. 75:3727-3731) and the tac promoter (DeBoer, et al., 1983, Proc. Natl. Acad. Sci. U.S.A. 80:21-25). Further references can also be found in "Useful proteins from recombinant bacteria" in Scientific American, 1980, 242:74-94.

According to one embodiment B. subtilis is transformed by an expression vector carrying the mutated DNA. If expression is to take place in a secreting microorganism such as B. subtilis a signal sequence may follow the translation initiation signal and precede the DNA sequence of interest. The signal sequence acts to transport the expression product to the cell wall where it is cleaved from the product upon secretion. The term "control sequences" as defined above is intended to include a signal sequence, when it is present.

EXAMPLES

SITE SPECIFIC MUTATION OF THE SUBTILISIN GENE GENERATES MUTANTS WITH USEFUL CHEMICAL CHARACTERISTICS

A number of the examples employ mutants which are not examples of the invention but illustrate the invention.

MATERIALS AND METHODS

BACTERIAL STRAINS

B. subtilis 309 and 147 are variants of Bacillus lentus, deposited with the NCIB and accorded the accession numbers NCIB 10147 and NCIB 10309, and described in U.S. Patent No. 3,723,250, issued March 27, 1973, and incorporated in its entirety by reference herein.

B. subtilis DN 497 is described in U.S. Serial No. 039,298 filed April 17, 1987, corresponding to EP Publ. No. 242 220, also incorporated by reference herein, and is an aro+ transformant of RUB 200 with chromosomal DNA from SL 438, a sporulation and protease deficient strain obtained from Dr. Kim Hardy of Biogen.

E. coli MC 1000 r⁻m+ (Casadaban, M.J. and Cohen, S.N. (1980), J. Mol. Biol. 138: 179-207), was made r⁻m+ by conventional methods and is also described in U.S. Serial No. 039,298.

B. subtilis DB105 is described in: Kawamura,F., Doi, R.H. (1984), Construction of a Bacillus subtilis double mutant deficient in extracellular alkaline and neutral proteases , J.Bacteriol. 160 (2), 442- 444.

PLASMIDS

pSX50 (described in U.S. patent application serial No. 039,298, and incorporated by reference herein) is a , derivative of plasmid pDN 1050 comprising the promoter-operator $P_1O_1$, the B. pumilus xyn B gene and the B. subtilis xyl R gene.

pSX62 (described in U.S. patent application serial No. 039,298, supra) is a derivative of pSX52 (ibid), which comprises a fusion gene between the calf prochymosin gene and the B. pumilus xyn B gene inserted into pSX50 (supra). pSX62 was generated by inserting the E. coli rrn B terminator into pSX52 behind the prochymosin gene.

pSX65 (described in U.S. patent application serial No. 039,298, supra) is a derivative of plasmid pDN 1050, comprising the promotor-operator $P_2O_2$, the B. pumilus xyn B gene, and the B. subtilis xyl R gene.

pSX88 (described in unpublished International Patent Application no. PCT/DK 88/00002 (NOVO INDUSTRI A/S)) is a derivative of pSX50 comprising the subtilisin 309 gene.

pSX92 was produced by cloning the subtilisin 309 into plasmid pSX62 (supra) cut at Cla I and Hind III, and Cla I filled prior to the insertion of the fragments DraI-NheI and NheI-Hind III from the cloned subtilisin 309 gene.

pSX93, shown in Figure 3, is pUC13 (Vieira and Messing, 1982, Gene 19::259-268) comprising a 0.7kb XbaI-Hind III fragment of the subtilisin 309 gene including the terminator inserted in a polylinker sequence.

pSX119 (described in unpublished International Patent Application no. PCT/DK 88/00002 (NOVO INDUSTRI A/S)) is pUC13 harbouring an EcoRI-XbaI fragment of the subtilisin 309 gene inserted into the polylinker.

pSX120 is a plasmid where the Hpal-HindIII fragment with the subtilisin 309 gene from pSX88 is inserted into EcoRV-HindIII on pDN 1681, in a way whereby the protease gene is expressed by the amy M and amy Q promotors. pDN 1681 is obtained from pDN 1380 (Diderichsen, B. and Christiansen, L.: 1988, FEMS Microbiology Letters 56: 53-60) with an inserted 2.85 bp ClaI fragment from B. amyloliquefaciens carrying the amy Q gene with promotor (Takkinen et al.: 1983, J. Biol. Chem. 258: 1007ff.)

pUC13 is described in: Vieira, J. and Messing, J.: 1982, Gene 19: 259-268.

pUC19 is described in: Yanisch-Perron, C. and Vieira, J. Messing, J., 1985, Gene 33:103-119.

pUB110 is described in: Lacey,R.W., Chopra,J. (1974), Genetic studies of a multiresistant strain of Staphylococcus aureus, J. Med.Microbiol. 7, 285-297, and in: Zyprian, E.. Matzura,H. (1986), Characterization of signals promoting gene expression on the Staphylococcus aureus plasmid pUB110 and development of a Gram-positive expression vector system, DNA 5 (3), 219-225.

GENES

The genes for the various subtilisins were obtained as referenced in the literature mentioned above. In particular the genes for the subtilisin 309 and 147 enzymes were obtained as described in unpublished international patent application no. PCT/DK 88/00002 (NOVO INDUSTRI A/S), which is hereby incorporated by reference in its entirety.

SUBTILISIN CARLSBERG GENE CONSTRUCTION

A synthetic gene was designed based on the coding sequence of the mature subtilisin Carlsberg protease and its transcription terminator (Jacobs, M. Eliasson,M., Uhlen,M., Flock,J.-I. (1985), Cloning, sequencing and expression of subtilisin Carlsberg from Bacillus licheniformis. Nucleic Acids Res. 13 (24), 8913-8926), linked to the pre and pro coding sequences of the subtilisin BPN' protease (Wells,J.A., Ferrari,E., Henner,D.J., Estell,D.A., Chen,E.Y. (1983), Cloning, sequencing and secretion of Bacillus amyloliquefaciens subtilisin in Bacillus subtilis, Nuclei Acids Res. 11 (22), 7911-7925). The gene was subdivided into seven fragments in length ranging from 127 to 313 basepairs, each fragment built up from chemically synthesized oligos of 16 to 77 nucleotides. The overlap between the oligos of the two strands was optimised in order to facilitate a one step annealing of each fragment (Mullenbach, G.T., Tabrizi, A., Blacher, R.W., Steimer,K.S. (1986), Chemical synthesis and expression in Yeast of a gene encoding connective tissue activating peptide-III, J.Biol. Chem. 261 (2), 719-722). Each fragment was assembled and cloned in an E, Coli cloning and sequencing vector. Sequence analysis of these cloned fragments was performed to confirm the correctness of the sequence of each fragment. Then all of the fragments were assembled and cloned in the vector pUB110 (Lacey,R.W., Chopra,J. (1974), Genetic studies of a multiresistant strain of Staphylococcus aureus, J. Med.Microbiol. 7, 285-297) and brought into B. subtilis DB105 (Kawamura,F., Doi, R.H. (1984), Construction of a Bacillus subtilis double mutant deficient in extracellular alkaline and neutral proteases , J.Bacteriol. 160 (2), 442- 444). Transcription of the gene was initiated by the HpaII promotor of the pUB110 plasmid vector (Zyprian,E., Matzura,H. (1986), Characterization of signals promoting gene expression on the Staphylococus aureus plasmid pUB110 and development of a Gram-positive expression vector system, DNA 5 (3), 219-225). In the process of the gene construction it turned out that the longest fragment (#5; 313 basepairs long) needed further fragmentation (fragments #8 and #9) in order to avoid problems with the assembly of this rather long fragment.

The amino acid sequence deduced from the nucleotide sequence differs from the earlier published subtilisin Carlsberg sequence at positions 129, 157, 161 and 212 (Smith,E.L., DeLange,R.J., Evans,W.H., Landon,W., Markland, F.S. (1968), Subtilisin Carlsberg V. The complete sequence: comparison with subtilisin BPN'; evolutionary relationships., J.Biol.-Chem. 243 (9), 2184-2191). A fifth alteration reported by Jacobs et al (1985) could not be confirmed in the clone of the Carlsberg gene described here.

COMPUTATION OF ISOELECTRIC POINT (pI$_o$)

The calculation of the isoelectric point of the subtilisin 309 wild type enzyme (S000) is exemplified below in order to demonstrate the procedure used. The same procedure is of course applicable to the computation of any enzyme, whether it being a mutant enzyme or not.

pK values were assigned to each potentially charged amino acid residue (Tyr, Asp, Glu, Cys, Arg, His, Lys, N-terminal, C-terminal, Ca$^{2+}$). In this case the environment was taken into consideration, whereby different pK values are used for the same amino acid residue dependent on its neighbours. The assigned values are indicated in Table II.

Then the ratio of the occurrence of an amino acid residue at a given pH in charged or uncharged form (charged/uncharged, C/U(i)) was calculated for both negative and positive charge, by using the formulae Ia and Ib, respectively. In Table II this ratio is only indicated for pH equal to pI$_o$.

Subsequently the relative charge, Q$_r$(i), or charge contribution allocated to each charged residue was calculated by using the formulae IIa and IIb:

The pH value where the sum of all the charge contributions from the charged residues is equal to zero was found by iteration.

Table II

Calculation of isoelectric point for: S000 Subtilisin 309

| Residue | Number of pK | Residue | C/U(i)* | Q$_r$(i) pH 8.3 | Q$_r$(i) pH 10.0 | Q$_r$(i) pH=pI$_o$ |
|---|---|---|---|---|---|---|
| Tyr | 9.9 | 3 | 2.51E-02 | -0.07 | -1.67 | -1.77 |
| Tyr | 11.6 | 2 | 5.01E-04 | 0.00 | -0.05 | -0.06 |
| Tyr | 12.5 | 2 | 6.31E-05 | 0.00 | -0.01 | -0.01 |
| Asp | 3.5 | 5 | 6.31E+04 | -5.00 | -5.00 | -5.00 |
| Glu | 4 | 5 | 2.00E+04 | -5.00 | -5.00 | -5.00 |
| C-term(Arg) | 3 | 1 | 2.00E+05 | -1.00 | -1.00 | -1.00 |
| Cys | 9.3 | 0 | 1.00E-01 | 0.00 | 0.00 | 0.00 |
| Arg | 12.8 | 8 | 3.16E+04 | 8.00 | 7.99 | 7.99 |
| His | 6.4 | 7 | 1.26E-02 | 0.09 | 0.00 | 0.00 |
| Lys | 10 | 5 | 5.01E+01 | 4.90 | 2.50 | 2.34 |
| Calcium | 20 | 1.25 | 5.01E+11 | 2.50 | 2.50 | 2.50 |
| N-term(Ala) | 8 | 1 | 5.01E-01 | 0.33 | 0.01 | 0.01 |
| | | | | | | |
| Net charge | | | | 4.75 | 0.27 | 0.0 |
| The calculated isoelectric point is | | | | 10.06 | | |

* E-02 = $10^{-2}$

As indicated above and in Table II the pK value assigned to each amino acid was different taking local variations in the environment into consideration. This only results in an enhanced precision in the calculation, but experience has shown that constant estimated pK values are helpful in showing in what direction the pI$_o$ for a given mutant enzyme will move in comparison to the pI$_o$ of the parent enzyme. This is indicated in Table III, where pI$_o$ values for estimated pK values are indicated.

In order to compare various enzymes and mutant enzymes washing tests described in detail below have been performed. In Table III below results from these tests using parent enzyme and mutant enzymes from subtilisin 309 (designated S000, etc.) and subtilisin Carlsberg (designated C000, etc.) have been tabulated in order to demonstrate the correlation between pI$_o$ and wash performance at different pH values of the wash liquor used. In the washing tests a low salt liquid detergent formulation of pH 8.3 according to detergent example D7, and a normal salt powder detergent of pH 10.2 according to detergent example D2 were used.

In Table III the results are indicated as relative results compared to the wild type enzymes (S000 and C000, respectively). Also, calculated and observed $pI_o$s for the enzymes are indicated.

Table III

Comparative washing tests at different pH values

| Mutant | $pI_o$ | | Improvement Factor | |
|--------|------------|----------|-----------|-------|
| | calculated | observed | Detergent 8.3 | pH 10.2 |
| S000 | 10.02 | 9.7 | 1 | 1 |
| S001 | 9.86 | 9.4 | 2.2 | 1 |
| S003 | 9.86 | 9.4 | 2.0 | 1 |
| S004 | 9.68 | 9.1 | 3.9 | 1 |
| S005 | 9.71 | 9.1 | 1.5 | 1 |
| S012 | 9.09 | 8.8 | 5.0 | 0.6 |
| S019 | 9.09 | 8.5 | 5.8 | 0.6 |
| S020 | 6.71 | 7.9 | 8.8 | 0.5 |
| S021 | 9.85 | - | 1.8 | 0.7 |
| S022 | 8.07 | - | 9.0 | 0.3 |
| S023 | 8.05 | - | 9.8 | 0.2 |
| S024 | 6.86 | - | 9.0 | 0.2 |
| S025 | 8.94 | - | 6.9 | 0.6 |
| S027 | 10.28 | - | 0.4 | 1.0 |
| S028 | 10.28 | - | 0.9 | 1.0 |
| S031 | 10.53 | - | 0.4 | 0.7 |
| S032 | 10.28 | - | 0.7 | - |
| S033 | 10.28 | - | 0.4 | - |
| S035 | 8.07 | - | 8.0 | 0.6 |
| S201 | 9.85 | - | 2.0 | 0.7 |
| S202 | 9.62 | - | 4.3 | 0.9 |
| S203 | 9.27 | - | 9.0 | 0.5 |
| C000 | 8.87 | - | 1 | 1 |
| C001 | 9.38 | - | 0.2 | 1.5 |
| C002 | 9.38 | - | 0.8 | 1.9 |
| C003 | 9.38 | - | 0.4 | 1.1 |
| C004 | 9.64 | - | 0.2 | 1.8 |
| C008 | 9.38 | - | 0.2 | 1.5 |

From Table III it is seen that shifting the $pI_o$ to lower values (S-series) provides for an improvement in wash performance at low pH (pH=8.3), whereas an upward shift in $pI_o$ (C-series) provides for an improvement in wash performance at high pH (pH=10.2).

The concept of isoelectric point has thus been found to be very useful in selecting the positions of the amino acids in the parent enzyme should be changed.

It has generally been found that mutations should be performed in codons corresponding to amino acids situated at or near to the surface of the enzyme molecule thereby retaining the internal structure of the parent enzyme as much as possible.

PURIFICATION OF SUBTILISINS

The procedure relates to a typical purification of a 10 litre scale fermentation of the Subtilisin 147 enzyme, the Subtilisin 309 enzyme or mutants thereof.

Approximately 8 litres of fermentation broth were centrifuged at 5000 rpm for 35 minutes in 1 litre beakers. The supernatants were adjusted to pH 6.5 using 10% acetic acid and filtered on Seitz Supra S100 filter plates.

The filtrates were concentrated to approximately 400 ml using an Amicon CH2A UF unit equipped with an Amicon S1Y10 UF cartridge. The UF concentrate was centrifuged and filtered prior to absorption at room temperature on a Bacitracin affinity column at pH 7. The protease was eluted from the Bacitracin column at room temperature using 25% 2-propanol and 1 M sodium chloride in a buffer solution with 0.01 dimethylglutaric acid, 0.1 M boric acid and 0.002 M calcium chloride adjusted to pH 7.

The fractions with protease activity from the Bacitracin purification step were combined and applied to a 750 ml Sephadex G25 column (5 cm dia.) equilibrated with a buffer containing 0.01 dimethylglutaric acid, 0.2 M boric acid and 0.002 m calcium chloride adjusted to pH 6.5.

Fractions with proteolytic activity from the Sephadex G25 column were combined and applied to a 150 ml CM Sepharose CL 6B cation exchange column (5 cm dia.) equilibrated with a buffer containing 0.01 M dimethylglutaric acid, 0.2 M boric acid, and 0.002 M calcium chloride adjusted to pH 6.5.

The protease was eluted using a linear gradient of 0-0.1 M sodium chloride in 2 litres of the same buffer (0-0.2 M sodium chloride in case of sub 147).

In a final purification step protease containing fractions from the CM Sepharose column were combined and concentrated in an Amicon ultrafiltration cell equipped with a GR81PP membrane (from the Danish Sugar Factories Inc.).

Subtilisin 309 and mutants

Gly 195 Glu (G195E (S001)):
Arg 170 Tyr (R170Y (S003)):
Arg 170 Tyr + Gly 195 Glu (R170Y+G195E (S004)):
Lys 251 Glu (K251E (5005)):
His 120 Asp (H120D (S006)):
Arg 170 Tyr + Gly 195 Glu + Lys 251 Glu
(R170Y+G195E+K251E (S012)):
Lys 235 Leu (K235L (S015)):
His 120 Asp + Gly 195 Glu + Lys 235 Leu (H120D+G195E+ K235L (S017)):
His 120 Asp + Arg 170 Tyr + Gly 195 Glu + Lys 235 Leu (H120D+R170Y+G195E+K235L (S019)) :
His 120 Asp + Arg 170 Tyr + Gly 195 Glu + Lys 235 Leu + Lys 251 Glu (H120D+R170Y+G195E+K235L+FC251E (S020): were purified by this procedure.

## PURIFICATION OF (MUTANT) SUBTILISIN CARLSBERG PROTEASES

Fermentation media were either directly applied on a bacitracin affinity column (5 cm diam * 15 cm; equilibrated with 10 mM Tris/HCl buffer pH 7.9; flow rate approx. 500 ml/h) or concentrated to 500 ml by means of a Nephross Andante H.F. dialyzer (Organon Technika) using a back pressure of 10-12 p.s.i. and demineralized water in the outer circuit. In the latter case the protease was precipitated from the concentrate by adding 600 g/l ammonium sulphate. The precipitate was collected by means of centrifugation and redissolved in approx. 500 ml demineralized water. The ammonium sulphate was removed from the protease solution using the same dialyzer as described above. The final volume was approx. 300 ml, while the pH was adjusted to pH 6.0. The protease was eluted from the bacitracin columns (mentioned above) using a 10 mM Tris buffer (pH 7.9) containing 2.7 M NaCl and 18% isopropanol.

After dialysis of bacitracin-purified or concentrated protease material further purification was accomplished by application on a CM-Trisacryl ion exchange column (5 cm. diam * 15 cm; equilibrated with 0.03M sodium phosphate pH 6.0) using a flow rate of 200 ml/h. The protease was eluted from the column with a linear gradient from 0 to 0.3 M NaCl (2 * 500 ml) in the phosphate buffer. Fractions containing protease activity were pooled and stored at -20°C in the presence of buffer salts after freeze-drying.

## OLIGONUCLEOTIDE SYNTHESIS

All mismatch primers were synthesized on an Applied Biosystems 380 A DNA synthesizer and purified by polyacrylamide gel electrophoresis (PAGE).

## ASSAY FOR PROTEOLYTIC ACTIVITY

The proteolytic activity of the mutant enzymes was assayed in order to determine how far the catalytic activity of the enzyme was retained. The determinations were performed by the dimethyl casein (DMC) method described in NOVO Publication AF 220-gb (or later editions), available from Novo-Nordisk a/s, Bagsværd, Denmark, which publication is hereby included by reference.

ASSAYS FOR WASH PERFORMANCE

A:

Test cloths (2.2cm x 2.2cm), approximately 0.1 g) were produced by passing desized cotton (100% cotton, DS 71) cloth through the vessel in a Mathis Washing and Drying Unit type TH (Werner Mathis AG, Zurich, Switzerland) containing grass juice.

Finally the cloth was dried in a strong air stream at room temperature, stored at room temperature for 3 weeks, and subsequently kept at -18° C prior to use.

All tests were performed in a model miniwash system. In this system 6 test cloths are washed in a 150 ml beaker containing 60 ml of detergent solution. The beakers are kept in a thermostat water bath at 30° C with magnetic stirring..

As detergent the following standard liquid detergent was used:

| | |
|---|---|
| AE, Berol 160 | 15% |
| LAS, Nasa 1169/P | 10% |
| Coconut fatty acid | 9% |
| Oleic acid | 1% |
| Triethanolamine | 9% |
| Glycerol | 10.5% |
| Ethanol | 1.5% |
| Tri$\times$Na$\times$Citrat$\times$2H$_2$O | 8% |
| CaCl$\times$2H$_2$O | 0.1% |
| NaOH | 1% |
| Water from LAS | 23.3% |
| Water from glycerol | 1.5% |
| Water added | 34.9% |

The percentages given are the percentage of active content.

pH was adjusted with 1 N NaOH to 8.14. The water used was ca. 6° dH (German Hardness).

Tests were performed at enzyme concentrations of: 0, 1.0 mg enzyme protein/1 and 10.0 mg enzyme protein/ 1, and two independent sets of tests were performed for each of the mutants. The results shown in the following are means of these tests.

The washings were performed for 60 minutes, and subsequent to the washing the cloths were flushed in running tap-water for 25 minutes in a bucket.

The cloths were then air-dried overnight (protected against daylight) and the remission, R, determined on an ELREPHO 2000 photometer from Datacolor S.A., Dietkikon, Switzerland at 460 nm.

As a measure of the wash performance differential remission, delta R, was used being equal to the remission after wash with enzyme added minus the remission after wash with no enzyme added.

B:

The wash performance of various mutants was tested against grass juice stained cotton cloths according to the method described above.

2.0 g/l of a commercial US liquid detergent was used.

The detergent was dissolved in a 0,005 M ethanolamine buffer in ion-exchanged water. pH was adjusted to pH 8.0, 9.0, 10.0 and 11.0 respectively with NaOH/HCl.

The temperature was kept at 30° C isothermic for 10 min.

The mutants were dosed at 0.25 mg enzyme protein/l each.

C:

Washing tests using the detergent compositions exemplified in the detergent examples below were performed in a mini washer utilizing cotton based test cloths containing pigments, fat, and protein (casein). The conditions were:

a) 2 g/l detergent D3 in 6°fH (French hardness) water at pH 8.3, or

b) 5 g/l detergent D2 in 15°fH water at pH 10.2.

After rinsing and drying reflection at 460 nm was measured.

The improvement factor was calculated from a dose-response curve, and relates to the amount of enzyme needed for obtaining a given delta R value in comparison to the wild type enzyme in question (S000 and C000), meaning that an improvement factor of 2 indicates that only half the amount of enzyme is needed to obtain the same delta R value.

The results of these tests are shown in Table III above.

D:

Experimental tests of lipase stability were carried out for example using the following materials:

1 LU/ml Pseudomonas cepacia lipase was incubated in wash liquor of each of two types, O and W (described below). Aliquots were taken at intervals and tested for lipase activity. Parallel incubations were carried out without protease or with protease of various types as noted below, to test the effect of the protease on the retention of lipase activity. Wild-type proteases were tested at 20 Gu/ml, mutated proteases were tested at 0.5 microgram/ml.

DETERRENT COMPOSITIONS COMPRISTNG ENZYME VARIANTS

Detergent D1:

A detergent powder according to an embodiment of the invention containing phosphate builder is formulated to contain: total active detergent about 16%, anionic detergent about 9%, nonionic detergent about 6%, phosphate-containing builder about 20%, acrylic or equivalent polymer about 3.5%, (alternatively down to about 2%), perborate bleach precursor about 6-18%, alternatively about 15-20%, amino-containing bleach activator about 2%, silicate or other structurant about 3.5%, alternatively up to about 8%, enzyme of about 8 glycine units/mg activity, with alkali to adjust to desired pH in use, and neutral inorganic salt, and enzymes (about 0.5% each enzyme).

The anionic detergent is a mixture of sodium dodecyl-benzene sulphonate, alternatively sodium linear alkyl-benzene-sulphonate, 6%, and primary alkyl sulphate 3%. The nonionic detergent is an ethoxylate of an approx. C13-C15 primary alcohol with 7 ethoxylate residues per mole. The phosphate builder is sodium tripolyphosphate. The polymer is polyacrylic acid, alternatively acrylic/maleic copolymer. The perborate bleach precursor is sodium tetraborate tetrahydrate or monohydrate. The activator is tetra-acetylethylene-diamine. The structurant is sodium silicate. The neutral inorganic salt is sodium sulphate.

The enzymes comprise protease according to Mutant S001, alternatively protease S003, S004, S005, C001, C002, C003, C004, C005, C008, S015, S017, S021, S226, 5223, S224, or S225.

Detergent D1a:

A detergent powder according to an embodiment of the invention containing phosphate builder is formulated to contain: total active detergent about 15%, anionic detergent about 7%, nonionic detergent about 6%, phosphate-containing builder about 25%, acrylic or equivalent polymer about 0.5%, perborate bleach precursor about 10%, amino-containing bleach activator about 2%, silicate or other structurant about 6%, protease enzyme of about 8 glycine units/mg grade, with alkali to adjust to desired pH in use, and neutral inorganic salt, and enzymes (about 0.5% each enzyme).

The anionic detergent is sodium linear alkyl-benzene-sulphonate. The nonionic detergent is an ethoxylate of an approx. C13-C15 primary alcohol with 7 ethoxylate residues per mole or a mixture of this with the corresponding alcohol ethoxylated to the extent of 3 residues per mole. The phosphate builder is sodium tripolyphosphate. The perborate or peracid bleach precursor is sodium tetraborate tetrahydrate. The activator is tetra-acetyl-ethylene-diamine. The structurant is sodium silicate. The neutral inorganic salt is sodium sulphate. The enzymes comprise protease according to Mutant S001, alternatively S003, S004, S005, C001, C002, C003, C004, C005, C008, S015, S017, S021, or S226.

Detergent D2:

A detergent powder according to an embodiment of the invention containing zeolite builder is formulated to contain: total active detergent about 16%, anionic detergent about 9%, nonionic detergent about 6%, zeolite-containing builder about 20%, acrylic or equivalent polymer about 3.5%, perborate bleach precursor about 6-18%, amino-containing bleach activator about 2%, silicate or other structurant about 3.5%, alternatively down to about 2.5%, enzyme of about 8 (alternatively about 15) glycine units/mg grade, with alkali to adjust to desired pH in use, and neutral inorganic salt, and enzymes (about 0.5% each enzyme).

The anionic detergent is a mixture of sodium dodecyl-benzene sulphonate, alternatively sodium linear alkyl-benzene-sulphonate, 6% and primary alkyl sulphate 3%. The nonionic detergent is an ethoxylate of an approx. C13-C15 primary alcohol with 7 ethoxylate residues per mole. The zeolite builder is type A zeolite. The polymer is polyacrylic acid. The perborate bleach precursor is sodium tetraborate tetrahydrate or monohydrate. The activator is tetraacetyl-ethylenediamine. The structurant is sodium silicate. The neutral inorganic salt is sodium sulphate. The enzymes comprise protease according to Mutant S001, alternatively S003, S004, S005, C001, C002, C003, C004, C005, C008, S015, S017, S021, or S226..

Detergent D2a:

A detergent powder according to an embodiment of the invention containing zeolite builder is formulated to contain: total active detergent about 14%, anionic detergent about 7%, nonionic detergent about 7%, zeolite-containing builder about 25%, acrylic or equivalent polymer about 3%, perborate or peracid bleach precursor about 10%, amino-containing bleach activator about 2%, silicate or other structurant about 0.5%, enzyme of about 6 glycine units/mg grade, with alkali to adjust to desired pH in use, and neutral inorganic salt, and enzymes (about 0.5% each enzyme).
The anionic detergent is sodium linear alkyl-benzene-sulphonate, the nonionic detergent is a mixture of ethoxylates of an approx. C13-C15 primary alcohol with 7 and 3 ethoxylate residues respectively per mole. The zeolite builder is type A zeolite. The polymer is an acrylic/maleic copolymer. The perborate bleach precursor is sodium tetraborate monohydrate. The activator is tetra-acetylethylene-diamine. The structurant is sodium silicate. The neutral inorganic salt is sodium sulphate. The enzymes comprise protease according to Mutant S001, alternatively S003, S004, S005, C001, C002, C003, C004, C005, C008, S015, S017, S021, or S226..

Detergent D3:

An aqueous detergent liquid according to an embodiment of the invention is formulated to contain: Dodecylbenzene-sulphonic acid 16%, C12-C15 linear alcohol condensed with 7 mol/mol ethylene oxide 7%, monoethanolamine 2%, citric acid 6.5%, sodium xylenesulphonate 6%, sodium hydroxide about 4.1%, protease 0.5%, minors and water to 100%. The pH is adjusted to a value between 9 and 10. The enzyme is a protease according to Mutant S020, alternatively S019, S012, S004, S001, S003, S005, S015, S017, S021, S022, S025, S035, S201, S223-S226 or S235.

Detergent D4:

A nonaqueous detergent liquid according to an embodiment of the invention is formulated using 38.5% C13-C15 linear primary alcohol alkoxylated with 4.9 mol/mol ethylene oxide and 2.7 mol/mol propylene oxide, 5% triacetin, 30% sodium triphosphate, 4% soda ash, 15.5% sodium perborate monohydrate containing a minor proportion of oxoborate, 4% TAED, 0.25% EDTA of which 0.1% as phosphonic acid, Aerosil 0.6%, SCMC 1%, and 0.6% protease. The pH is adjusted to a value between 9 and 10, e.g. about 9.8. The enzyme comprises protease according to Mutant S001, alternatively S003, S004, S021, S035, S201, S225, S226, or S235.

Detergent D5:

A detergent powder according to an embodiment of the invention is formulated in the form of a granulate having a bulk density of at least 600 g/l, containing about 20% by weight surfactant of which about 10% is sodium dodecylbenzene sulphonate, and the remainder is a mixture of Synperonic A7 and Synperonic A3 (about 5.5% to 4.5%), and zero neutral inorganic salt (e.g. sodium sulphate), plus phosphate builder about 33%, sodium perborate tetrahydrate about 16%, TAED activator about 4.5%, sodium silicate about 6%, and minors including sodium carbonate about 2%, and moisture content about 10%. Enzymes (about 0.5% each enzyme) are included. The enzyme comprises protease according to Mutant S001, alternatively S003, S004, S005, C001, C002, C003, C004, C005, C008, S223, S224, S225, S226, or S235.

Detergent D6:

A detergent powder according to an embodiment of the invention is formulated in the form of a granulate having a bulk density of at least 600 g/l, alternatively about 550 g/l, containing about 20%, alternatively down to about 16%, by weight surfactant of which about 9%, alternatively about 7%, is sodium dodecylbenzene sulphonate, alternatively sodium linear alkyl benzene sulphonate, and the remainder is a mixture of Synperonic A7 and Synperonic A3 (or similar ethoxylates) (respectively about 5% & 6%, alternatively about 4% and 7%), and zero neutral inorganic salt (e.g. sodium sulphate), plus zeolite builder about 30%, alternatively about 25%, sodium perborate tetrahydrate, alternatively monohydrate, about 14% or 15%, TAED activator about 3.6%, and minors including sodium carbonate about 9%, or up to 15%, Dequest® 2047 about 0.7%, and moisture content about 10%. Enzymes (about 0.5% each enzyme, or about 0.2% lipase and about 0.7% protease) are included. The enzyme comprises protease according to Mutant S001, alternatively S003, S004, S005, C001, C002, C003, C004, C005, C008, S223, S224, S225, S226, or S235.

Detergent D6a:

A detergent powder according to an embodiment of the invention is formulated in the form of a granulate having a bulk density of at least 600 g/l, containing about 15% by weight surfactant of which about 7% is sodium linear alkyl benzene sulphonate, 2% primary alcohol sulphate, and the remainder Synperonic A7 or similar ethoxylate, and zero neutral inorganic salt (e.g. sodium sulphate), plus zeolite builder about 22%, sodium perborate tetrahydrate about 15%, TAED activator about 7%, and minors including sodium carbonate about 15%, Dequest® 2047 about 0.7%, and moisture content about 10%. Enzymes (about 1.2%) include protease according to Mutant S001, alternatively S003, S004, S005, C001, C002, C003, C004, C005, C008, S223, S224, S225, S226, or S235.

Detergent D7:

A detergent powder according to an embodiment of the invention is formulated to contain: Dodecylbenzenesulphonic acid 6%, C12-C15 linear alcohol condensed with 7 mol/mol ethylene oxide 5%, fatty acid soap 3%, Sokolan® CP5 polymer 3%, zeolite A 22%, sodium carbonate 10%, sodium sulphate 17%, clay particles 8%, sodium perborate tetrahydrate 13%, tetraacetyl-ethylenediamine 2%, protease 0.5%, minors and water to 100%. The pH is adjusted to a value between 9 and 10. The protease enzyme comprises protease according to Mutant S001, alternatively S003, S004, S005, C001, C002, C003, C004, C005, C008, S223, S224, 5225, S226, or S235.

Detergent D8:

A detergent (soap) bar according to an embodiment of the invention is formulated as follows: soap based on pansa-ponified 82% tallow, 18% coconut oil, neutralised with 0.15% orthophosphoric acid, mixed with protease (about 8 GU/mg of the bar composition) and mixed with sodium formate 2%, borax 2%, propylene glycol 2% and sodium sulphate 1%, is then plodded on a soap production line. The protease enzyme comprises protease according to Mutant S001, alternatively S003, S004, S005, C001, C002, C003, C004, C005, C008, S021, S025, S035, S201, S202, S223, S224, S225, S226, or S235.

Detergent D9:

Structured liquid detergents can for example contain, in addition to a protease as described herein, 2-15% nonionic surfactant, 5-40% total surfactant, comprising nonionic and optionally anionic surfactant, 5-35% phosphate-containing or non-phosphate containing builder, 0.2-0.8% polymeric thickener, e.g. cross-linked acrylic polymer with m.w. over 106, at least 10% sodium silicate, e.g. as neutral waterglass, alkali (e.g. potassium-containing alkali) to adjust to desired pH, preferably in the range 9-10 or upwards, e.g. above pH 11, with a ratio sodium cation: silicate anion (as free silica) (by weight) less than 0.7:1, and viscosity of 0.3-30 Pas (at 20°C and 20s-1).
Suitable examples contain about 5% nonionic surfactant C13-15 alcohol alkoxylated with about 5 EO groups per mole and with about 2.7 PO groups per mole, 15-23% neutral waterglass with 3.5 weight ratio between silica and sodium oxide, 13-19% KOH, 8-23% STPP, 0-11% sodium carbonate, 0.5% Carbopol® 941.
Protease may be incorporated at for example 0.5% of Protease according to Mutant S001, alternatively S021, S025, S035, S201, S202, S223, S224, S225, S226, or S235.

Detergent D10

A structured, viscous, aqueous liquid detergent suitable for laundry use is formulated as follows (% by weight):

| | |
|---|---|
| Citric acid | 2.5 |
| Borax (10aq) | 4 |
| NaOH | 2 |
| Glycerol | 5 |
| C14-C15 Linear alkyl-benzene-sulphonate, or C14-15 primary alcohol sulphate Synperonic A3 | 6.5 |
| Nonionic C12-C15 3EO | 1.2 |

(continued)

| | |
|---|---|
| Synperonic A7 | |
| Nonionic C12-C15 7EO | 3.6 |
| Zeolite | 20 |
| Protease | 0.5 |
| Amylase (Termamyl® 300LDX) | 0.2 |
| minors and water | to 100% |

The pH can be adjusted to a value between 9 and 10. The enzyme is a protease according to Mutant S020. In alternative versions of this Detergent, the enzyme is selected from S019, S012, S004, S001, S003, S005, S021, S035, S201, S223, S224, S225, S226, or S235.

Detergent D11

An isotropic aqueous liquid detergent suitable for laundry use is formulated as follows (% by weight):

| | |
|---|---|
| Citric acid | 2 |
| Boric acid | 1 |
| NaOH | 3 |
| KOH | 4.5 |
| Glycerol | 10 |
| Ethanol | 6.5 |
| Nonionic surfactant (C12-alcohol 6.5 EO ethoxylate groups/mol) or sodium primary alcohol sulphate | 10 |
| Oleic acid | 16 |
| Coconut oil (C12) soap | 11 |
| Protease | 0.5 |
| minors and water | to 100% |

The pH can be adjusted to a value between 9 and 10. The enzyme is a protease according to Mutant S020. In alternative versions of this Detergent, the enzyme is selected from S019, S012, S004, S001, S003, S005, S021, S025, S035, S201, S223, S224, S225, S226, or S235.

Detergent D12

An aqueous liquid detergent composition is formulated to contain:

| | |
|---|---|
| sodium alkyl-benzene-sulphonate | 14.5 |
| C18 sodium soap | 2 |
| Nonionic detergent (C12-15 6EO) | 9 |
| Fatty acid (oleic acid) | 4.5 |
| sodium alkenyl succinate | 11 |
| propanediol | 1.5 |
| ethanol | 3.6 |
| sodium citrate | 3.2 |
| Complexing agent e.g. Dequest 2060 | 0.7 |
| Protease | 0.5 |
| Amylase | 0.1 |
| Sodium chloride | 0.5 |
| minors and water | to 100% |

The pH can be adjusted to a value between 9 and 10. The enzyme is a protease according to Mutant S020. In alternative versions of this Detergent, the enzyme is selected from S019, S012, S004, S001, S003, S005, S021, S025, S035, S201, S202, S223, S224, S225, S226, or S235.

Detergent D13

An aqueous liquid detergent composition is formulated to contain:

| | |
|---|---|
| sodium alkyl-benzene-sulphonate | 8 |
| nonionic detergent 6.5EO | 10 |
| Oleic diethylamide | 10 |
| Fatty acid (C12/C18 75:25) | 18 |
| sodium citrate | 1 |
| triethanolamine | 5 |
| propanol | 7 |
| ethanol | 5 |
| Dequest 2060 | 0.5 |
| Protease | 0.5 |
| Amylase | 0.1 |
| minors and water | to 100% |

The pH can be adjusted to a value between 9 and 10. The enzyme is a protease according to Mutant S020. In alternative versions of this Detergent, the enzyme is selected from S019, S012, S004, S001, S003, S005, S021, S025, S035, S201, S202, S223, S224, S225, S226, or S235.

Detergent D14

A non-aqueous liquid detergent composition is formulated to contain (% by weight) :

| | |
|---|---|
| Liquid nonionic detergent (C10-12, 6.2EO) | 41% |
| triacetin | 5 |
| linear alkylbenzenesulphonic acid | 6 |
| magnesium oxide stabiliser | 1 |
| Sodium carbonate builder/base | 18 |
| Calcium carbonate builder | 8 |
| bleach activator TAED | 3.5 |
| bleach precursor perborate monohydrate | 10.5 |
| partly-hydrophobic silica | 2 |
| protease | 0.4 |
| lipase (Lipolase®) | 3 |
| minors or additional | |
| liquid nonionic surfactant (no water) | to 100% |

In formulating this composition, the liquid nonionic surfactant and triacetin are added first, followed by the magnesium oxide, then the other ingredients except enzyme. The mixture is milled in a colloid mill and cooled, and finally the enzyme(s) and any other heat-sensitive minors are added.
The enzyme is a protease according to Mutant S020. In alternative versions of this Detergent, the enzyme is selected from S019, S012, S004, S001, S003, S005, S021, S025, S035, S201, S202, S223, S224, S225, S226, or S235.
Also usable are any one of the detergent formulations described and exemplified in EP 0 342 177 in conjunction with mutants as for Detergent D3.

<u>RESULTS</u>

<u>GENERATION OF SITE-SPECIFIC MUTATIONS OF THE SUBTILISIN 309 GENE</u>

Site specific mutations were performed by the method of Morinaga et al. (Biotechnology, <u>supra</u>). The following oligonucleotides were used for introducing the mutations:

31

a) <u>Gly 195 Glu (G195E (S001))</u>:
A 27-mer mismatch primer, Nor-237, which also generates a novel SacI restriction site:

```
              5'   CACAGTATGGGCGCAGGGCTTGACATTGTCGCACCAGG   3'
Nor-237       5'   GTATGGCGCAGAGCTCGACATTTGTCGC             3'
                             SacI
```

b) <u>Arg 170 Tyr (R170Y (S003))</u>:
A 25-mer mismatch primer, Nor-577, which destroys a HaeIII site:

```
                        HaeIII
              5'   GCTATCCGGCCCGTTATGCGAACGC   3'
Nor-577       3'   CGATAGGCCGTATAATACGCTTGCG   5'
```

c) <u>His 120 Asp (H120D (S006))</u>:
A 32-mer mismatch primer, Nor-735, which destroys a SphI site:

```
                      SphI
              5'   AGGGAACAATGGCATGCACGTTGCTAATTTGA   3'
Nor-735       5'   AGGGAACAATGGCATGGACGTTGCTAATTTGA   3'
```

d) <u>Lys 251 Glu (K251E (S005))</u>:
A 32-mer mismatch primer, Nor-736, which generates a XhoI site:

```
              5'   CAAATCCGCAATCATCTAAAGAATACGGCAAC   3'
Nor-736       5'   CAAATCCGCAATCATCTCGAGAATACGGCAAC   3'
                                   XhoI
```

e) <u>Lys 235 Leu (K235L (S015))</u>:
A 24-mer mismatch primer, Nor77-856, which generates a PStI site:

```
              5'   GCCCTTGTTAAACAAAAGAACCCA   3'
Nor-856       5'   GCCCTTGTTCTGCAGAAGAACCCA   3'
                            PstI
```

f) <u>Arg 170 Tyr; Gly 195 Glu (R170Y;G195E (S004))</u>:
A combination of Nor-577 and Nor-237 was performed in analogy with the above.
g) <u>Gly 195 Glu; 251 Glu (G195E;K251E (S018))</u>:
A combination of Nor-237 and Nor-736 was performed in analogy with the above.
h) <u>Arg 170 Tyr; Lys 251 Glu (R170Y;K251E (S011))</u>:
A combination of Nor-577 and Nor-736 was performed in analogy with the above.
i) <u>Arg 170 Tyr: Gly 195 Glu; Lys 251 Glu (R170Y;G195E;K251E (S012))</u>:
A combination of Nor-577, Nor-237, and Nor-736 was performed in analogy with the above.
j) <u>Gly 195 Glu; Lys 235 Leu (G195E;K235L)</u>:
A combination of Nor-237 and Nor-856 was performed in analogy with the above.
k) <u>Arg 170 Tyr; Gly 195 Glu; Lys 235 Leu (R170Y;G195E;K235L)</u>:
A combination of Nor-577, Nor-237, and Nor-856 was performed in analogy with the above.
1) <u>His 120 Asp; Lys 235 Leu (H120D;K235L (S016))</u>:

A combination of Nor-735 and Nor-856 was performed in analogy with the above.

m) His 120 Asp; Gly 195 Glu; Lys 235 Leu (H120D;G195E;K235L (S017)):

A combination of Nor-735, Nor-237, and Nor-856 was performed in analogy with the above.

n) His 120 Asp; Arg 170 Tyr; Gly 195 Glu; Lys 235 Leu (H120D;R170Y;G195E;K235L (S019)) :

A combination of Nor-735, Nor-577, Nor-237, and Nor-856 was performed in analogy with the above.

o) His 120 Asp; Arg 170 Tyr; Gly 195 Glu; Lys 235 Leu; Lys 251 Glu (H120D;R170Y;G195E;K235L;K251E (S020):

A combination of Nor-735, Nor-577, Nor-237, Nor-856, and Nor-736 was performed in analogy with the above.

Gapped duplex mutagenesis was performed using the plasmids pSX93, pSX119, and pSX120 as templates.

pSX93 is shown in Figure 3 and is pUC13 (Vieira, J. and Messing, J.: 1982, Gene 19: 259-268) harbouring an 0.7 kb XbaI-HindIII fragment of the subtilisin 309 gene including the terminator inserted in the polylinker.

For the introduction of mutations in the N-terminal part of the enzyme the plasmid pSX119 was used. pSX119 is pUC13 harbouring an EcoRI-XbaI fragment of the subtilisin 309 gene inserted into the polylinker. The templates pSX 93 and pSX119 thus cover the whole of the subtilisin 309 gene.

Plasmid pSX120 is a plasmid where the HpaI-HindIII fragment with the subtilisin 309 gene from pSXB8 is inserted into EcoRV-HindIII on pDN 1681, in a way whereby the protease gene is expressed by the amy M and amy Q promotors. pDN 1681 is obtained from pDN 1380 (Diderichsen, B. and Christiansen, L.: 1988, FEMS Microbiology Letters 56: 53-60) with an inserted 2.85 bp ClaI fragment from B. amyloliquefaciens carrying the amy Q gene with promotor (Takkinen et al.: 1983, J. Biol. Chem. 258: 1007ff.). The construction of pSX120 is outlined in Fig. 1, showing that pDN1681 is cut with EcoR5 and HindIII, and pSX88 with HindIII and HpaI, whereafter ligation results in pSX120 regulated by the amy M and amy Q promotors.

Four further plasmids pSX170, pSX172, pSX173, and pSX186 were constructed for gapped duplex mutagenesis of the subtilisin 309 gene:

- pSX170: SphI-KpnI, 700 bp from pSX120 inserted into pUC 19 SphI-KpnI, from amino acid residue 170 in mature subtilisin 309 to terminator.
- pSX172: EcoRI-SphI, 1360 bp from pSX120 inserted into pUC 19 EcoRI-SphI, from the promoter to amino acid residue 170 in mature subtilisin 309.
- pSX173: like pSX170, but with G195E.
- pSX186: PvuII-EcoRI, 415 bp from pSX120 inserted into pUC 19 HincI-EcoRI, from amino acid residue 15 to amino acid residue 206 in mature subtilisin 309.

Figure 2 shows a somewhat detailed restriction map of pSX120 on which it is indicated which fragments were used for the construction of plasmids pSX170, pSX172, pSX173, and pSX186.

The mutation a) was performed by cutting pSX93 with XbaI and ClaI as indicated in Figure 3 and described in the section "GENERATION OF SITE DIRECTED MUTATIONS IN THE SUBTILISIN GENE" and in unpublished International Patent Application no. PCT/DK 88/00002 (NOVO INDUSTRI A/S)

Mutations b), d) and e) were performed correspondingly by cutting pSX170 by SphI and KpnI.

Mutations f) and g) were performed as above, but with pSX173 in stead of pSX170.

Mutation c) was performed correspondingly by cutting pSX186 by PstI and EcoRI.

The mutations h) to o) were constructed by combining DNA fragments with single or double mutations b) to g) using the restriction sites NheI, XbaI, ClaI, AvaII, and KpnI as appropriate.

Further mutants were produced using similar methods or general methods as known from the literature.

SUBTILISIN CARLSBERG MUTANTS

For certain examples of mutations in subtilisin Carlsberg mentioned in this specification the following changes in the nucleotide sequence of the gene were introduced:

Asp 14 Lys (D14K (C001)) (GAT -> AAG)
Asp 120 Lys (D120K (C002)) (GAT -> AAA)
Asp 140 Lys (D140K (C003)) (GAC -> AAA)
Asp 14 Lys + Asp 120 Lys (D14K+D120K (C004))
Lys 27 Asp (K27D (C005)) (AAA -> GAT)
Lys 27 Asp + Asp 120 Lys (K27D+D120K (C006))
Asp 172 Lys (D172K (C008)) (GAC -> AAA)
Asp 14 Lys + Asp 120 Lys + Asp 140 Lys + Asp 172 Lys
(D14K+D120K+D140K+D172K (C010))

Val 51 Asp (V51D (C100))
Glu 54 Thr (E54T (C101)) (GGG -> ACA)
Glu 54 Tyr (E54Y (C102)) (GGG -> TAT)

These changes were introduced by changing the corresponding oligos in the fragments concerned. The correctness of the new sequences was confirmed after which the original oligos were replaced by these new sequences and assembled into new DNA fragments. Finally the fragments were re-assembled into the new subtilisin Carlsberg gene.

EXPRESSION OF MUTANT SUBTILISINS

Subsequent to sequence confirmation of the correct mutation the mutated DNA fragments were inserted into plasmid pSX92 or pSX120, which were used for producing the mutants.

Plasmid pSX92 is shown in Figure 4 and was produced by cloning the Sub 309 gene into plasmid pSX62 cut at ClaI, filled in with the Klenow fragment of DNA polymerase I and cut with HindIII prior to the insertion of the fragments DraI-NheI and NheI-HindIII from the cloned Sub 309 gene.

To express the mutants the mutated fragments (XbaI-ClaI, XbaI-HindIII, or EcoRI-XbaI) were excised from the appropriate mutation plasmid pSX93, pSX119, pSX170, pSX172, pSX173, and pSX186, respectively, and inserted into pSX92, or pSX120 to obtain plasmids capable of expressing the various mutants.

The mutated pSX92 or pSX120 were then used to transform B. subtilis DN497.

The transformed cells were then spread on LB agar plates with 10 mM phosphate, pH 7, 6mg/ml chloramphenicol, and 0.2% xylose to induce the xyn-promoter in the plasmid. The plates also contained 1% skim milk so the protease producing transformants could be detected by the clear halo where the skim milk had been degraded.

After appropriate growth the mutated enzymes were recovered and purified.

FERMENTATION OF THE SUBTILISIN CARLSBERG SPECIES

In order to produce protease enzyme on the basis of the microorganisms carrying mutant genes for BPN' as described above, a Rushton-type Chemoferm fermenter was generally used with an eight flat blade impeller and a working volume of 8 litres. The fermenter configuration was prepared conform to the safety regulations for VMT and consisted of:

a) A pressure controller (type 4-3122, Bell & Howell) cutting off air supply above 0.1 bar overpressure. This is done to prevent clogged exhaust air filters.
b) A foam trap on the gas outlet made from a 20 l suction vessel having anti-foam on the bottom.
c) A cooling water jacket without seals in order to prevent contamination of the cooling water or tap-water drain.
d) An absolute exhaust filter is used (Gelman acro 50, 0.45 micron).
e) Sampling via a sampling pump device with a small internal volume.

Controls

Gas flows were controlled using mass-flow meters (Brooks, type 5852, range 0-10 l).

pH was controlled using a Hartmann and Braun transmitter and a Philips controller (Witromat). Concentrated NaOH (3M) was used as a neutralizer.

Exhaust gases were analyzed using a Unor 4N (CO2) and an Oxygor 7N (02) from Maihak, Westinghouse. Oxygen tension in the medium was determined using an industrial polarographic sterilizable oxygen probe (Ingold type 322756702).

The medium temperature was monitored using a PT100 sensor and a Honeywell temperature controller (class 84). Foaming was kept at an acceptable level using a contact electrode, while a level switch activated an anti-foam dosage pump.

All external controls were put under the control of a Hewlett Packard microcomputer (HP220).

Cultivation conditions

The inocula were prepared by incubating a shake flask culture at 30°C for 16h at 250 rpm in a rotary shaker (LH fermentation, type MKx). 300 ml inoculum was used for 8L medium being conditioned at the actual fermentation conditions (pH 7.0, 30°C, air flow 3.5 l/min, stirrer 1000-1500 rpm). Dissolved oxygen concentration was kept 25% above air saturation. Anti foaming agent used was a silicon oil based material (Rhodorsil R426, Rhone Poulenc).

Production of subtilisin protease

The (mutant) proteases were produced using the <u>B. subtilis</u> DB105 strain containing the mutant gene as described under gene construction. The culture medium consists of : 8 g/l $NH_4Cl$; 4 g/l $KH_2PO_4$; 4 g/l $K_2HPO_4$; 2 g/l NaCl; 1 g/l $MgSO_4.2H_2O$; 10 g/l yeast extract; 40 g/l sucrose;; pH 7.0 and sterilized 45 min at 120°C. After sterilization 25 mg/l tryptophan; 20 mg/l Neomycin were added. Fermentations were stopped after 20 - 30 hours. The media were cleared from cells by centrifugation.

PROTEOLYTIC ACTIVITY OF MUTANT SUBTILISINS

The proteolytic activity of various mutants was tested against casein as protein substrate, according to the DMC method <u>supra</u>. The results are presented in Table IV.

From the table it is seen that mutant (S005) exhibits a slightly enhanced activity compared to the parent (S000), whereas the remaining mutants exhibit a slightly decreased activity.

TABLE IV

Proteolytic Activity of Mutant Subtilisins

| Mutant | Relative Activity |
|---|---|
| None (S000) | 100 |
| S001 | 95 |
| S003 | 90 |
| S004 | 85 |
| S005 | 105 |
| S006 | 100 |
| S012 | 80 |
| S017 | 90 |
| S019 | 70 |
| S020 | 75 |
| S024 | 70 |

WASH PERFORMANCE OF MUTANT SUBTILISINS

A:

The wash performance of various mutants in the standard liquid Detergent of pH 8.14 was tested in a model system against grass juice according to the methods detailed <u>supra</u>. The results are presented in table V.

Table V

Delta R values:

| Mutant | Enzyme Concentration | |
|---|---|---|
| | 1.0 mg/l | 10.0 mg/l |
| S000 | 4.0 | 10.7 |
| S001 | 5.9 | 12.8 |
| S003 | 6.0 | 13.5 |
| S004 | 5.8 | 13.0 |
| S012 | 4.2 | 9.6 |
| S019 | 10.5 | 19.4 |
| S020 | 9.4 | 18.6 |

From the table it is seen that all of the tested mutants exhibited improved or equal wash performance compared to the wild type parent enzyme. The wash performance of the mutants S019 and S020 is improved so that 1.0 mg/l of these enzymes roughly stated should be able to replace 10.0 mg/l of the wild type parent enzyme, thereby indicating a substantial improvement in the wash performance for the mutant enzymes of the invention.

B:

The results from tests of some of the enzyme variants of the invention in the modified commercial US liquid detergent at various pH values in a model system are shown in Table VI.

Table VI
Wash performance at different pH's

| Mutant | | Delta R | | | |
|--------|---------|------|------|------|------|
| | $pI_o$ pH | 8.0 | 9.0 | 10.0 | 11.0 |
| S000 | 10.02 | 1.4 | 2.6 | 3.1 | 10.1 |
| S001 | 9.86 | 2.1 | 4.0 | 6.6 | 14.0 |
| S003 | 9.86 | 2.3 | 5.0 | 8.1 | 14.1 |
| S004 | 9.68 | 4.1 | 9.7 | 11.7 | 10.9 |
| S005 | 9.71 | 2.2 | 4.3 | 6.3 | 13.9 |
| S012 | 9.09 | 5.7 | 11.9 | 13.8 | 6.3 |
| S019 | 9.09 | 6.4 | 10.7 | 12.2 | 3.7 |
| S020 | 6.71 | 7.8 | 10.6 | 8.5 | 2.4 |

The results show clearly, that shifting the pI of an enzyme in a direction where it is desired to shift the pH optimum for the wash performance of the enzyme to approach the pH of the wash liquor improves the wash performance of the enzyme.

D:

The wash performance of various mutants was tested against grass juice stained cotton cloths according to the method described in Assay A.

2.0 g/l of a liquid detergent (Detergent D3) was used. The detergent was dissolved in ion-exchanged water. pH was adjusted to 9.1 with NaOH/HCl.

The temperature was kept at 20°C isothermic for 10 min.

The mutants was dosed at 0.25; 0.5; 1.0; 2.0; 5.0; and 10.0 mg enzyme protein/l each.

The stability of the mutants tested was determined by measuring the denaturation temperature (maximum excess heat capacity) by differential scanning calorimetry, DSC. The heating rate was 0.5°C/min.

The stability was tested in a solution containing approx. 2 mg/ml of the mutant in 91% standard liquid detergent, the composition of which is described in Assay A. The solution was made by mixing 100 ml of enzyme solution (approx. 20 mg enzyme/ml in a buffer of 0.01 M dimethylglutaric acid, 0.002 M $CaCl_2$, 0.2 M $H_3BO_3$ and 0-0.1 M NaCl pH 6.5) with 1000 ml standard liquid detergent.

Within the group of Subtilisin 309 mutants stability results obtained by DSC are consistent with stability results obtained by traditional storage stability tests.

Results

The wash performance of various mutants in liquid detergent is presented in Table VII. The results are shown as improvement factors relative to the wild type parent enzyme. The improvement factor is defined as in Assay C.

Also shown in Table VII is the denaturation temperature in standard liquid detergent by DSC, and the difference between the denaturation temperature of the wild type patent enzyme and that of the mutant in question.

Table VII

| mutant | $pI_o$ calculated | Improvement factor | Denaturation temperature by DSC (°C) | Denaturation temperature by DSC relative to S000 |
|--------|-------------------|--------------------|--------------------------------------|--------------------------------------------------|
| S 000 | 10.06 | 1 | 65.2 | 0.0 |
| S 020 | 7.30 | 7.6 | 58.2 | -7.0 |
| S 021 | 9.85 | 1.3 | 69.2 | +4.0 |
| S 022 | 8.07 | 9.3 | 61.9 | -3.3 |
| S 023 | 8.05 | 8.8 | 63.5 | -1.7 |
| S 024 | 6.86 | 3.9 | 60.6 | -4.6 |

(continued)

| mutant | pI$_o$ calculated | Improvement factor | Denaturation temperature by DSC (°C) | Denaturation temperature by DSC relative to S000 |
|---|---|---|---|---|
| S 025 | 8.94 | 6.7 | 69.1 | +3.9 |
| S 035 | 8.07 | 7.0 | 72.5 | +7.3 |
| S 201 | 9.85 | 1.4 | 69.4 | +4.2 |

From Table VII it is seen that all of the tested mutants exhibit improved wash performance compared to the wild type parent enzyme. The best wash performance is achieved by the mutants having pI$_o$ equal to or just below the pH of the wash solution.

Denaturation temperature by DSC shows that the stability of the single mutants S 021 (*36D) and S 201 (N76D) is increased by 4.0°C and 4.2°C respectively relative to the wild type parent enzyme.

Among the mutations that are incorporated in one or more of the mutants listed in Table VII it has been shown that the mutations R170Y and K251E destabilize the mutant relative to the wild type parent enzyme, whereas the mutations H120D, G195E and K235L is indifferent with respect to stability.

It is seen from Table VII that mutants containing one destabilizing mutation are destabilized, even in cases, where a stabilizing mutation is included.

The stabilizing effects of *36D and N76D are additive. This is shown by the mutants S 025 and S 035. S 025 contains three mutations which are indifferent to stability and the stabilizing mutation *36D. The denaturation temperature for S 025 is increased by 3.9°C relative to the wild type parent enzyme, which is equal to the increase measured for the single mutant *36D, S 021. S 035 contains the same mutation N76D. The denaturation temperature for S 035 is increased by 7.3°C relative to the wild type parent enzyme, which, within experimental error, is equal to the sum of the increase measured for the single mutants *36D, S 021 and N76D, S 201.

E:

The wash performance of three mutants was tested against grass juice stained cotton cloth according to the method described in Assay A.

2.0 g/l of liquid detergent D3 was used. The detergent was dissolved in ion-exchanged water. pH was adjusted to 9.1 with NaOH/HCl.

The temperature was kept at 30°C isothermic for 10 min. The mutants were dosed at 1.0 and 10.0 mg enzyme protein/l each.

Results

The wash performance of three mutants in commercial US-liquid detergent was tested against grass juice. The results are shown in Table VIII.

Table VIII

Delta R values:

| Mutant mg/l | calculated pI$_o$ | Enzyme concentration 1.0 mg/l | 10.0 |
|---|---|---|---|
| S 000 | 10.06 | 4.5 | 13.6 |
| S 003 | 9.75 | 9.4 | 18.0 |
| S 004 | 9.54 | 13.7 | 18.1 |
| S 006 | 9.85 | 6.0 | 15.6 |

From Table VIII it is seen that all of the mutants exhibit improved wash performance relative to the wild type parent enzyme. It is further seen that the best performance is achieved by the mutant having pI$_o$ closest to the pH of the wash solution.

F:

The wash performance of two mutants was tested against grass juice stained cotton cloth according to the conditions described in Example E.

Results

The wash performance of two mutants in detergent D3 was tested against grass juice stained cotton cloth. The results are shown in Table IX.

Table IX

Delta R values:

| Mutant | calculated $pI_o$ | Enzyme concentration 1.0 mg/l | 10.0 mg/l |
|---|---|---|---|
| S 000 | 10.06 | 5.8 | 15.3 |
| S 015 | 9.95 | 8.4 | 20.0 |
| S 017 | 9.40 | 17.0 | 20.8 |

Prom Table IX it is seen that all of the mutants exhibit improved wash performance relative to the wild type parent enzyme. It is further seen that the best performance is achieved by the mutant having $pI_o$ closest to the pH of the wash solution.

G:

The wash performance of various mutants was tested on grass juice stained cotton cloth according to the method described in Assay A.
2.0 g/l of detergent D3 was used.
The detergent was dissolved in buffer (0,0025 M Boric acid and 0,001 M disodium hydrogen phosphate prepared in ion exchanged water). pH was adjusted to 7.0 , 8.0 , 9.0 , and 10.0 respectively with NaOH/HCl. The temperature was kept at 30°C isothermic for 10 min.
The mutants were dosed at 0,2 mg enzyme protein/l each.

Results:

The wash performance of some of the enzyme variants of the invention at various pH values in a model system are shown in table X.

Table X

| Variant | Mutation | $pI_0$ | Delta R pH 7.0 | 8.0 | 9.0 | 10.0 |
|---|---|---|---|---|---|---|
| S000 | | 10.06 | 0.6 | 0.8 | 4.4 | 7.0 |
| S015 | .K235L | 9.95 | 1.3 | 2.4 | 6.0 | 8.8 |
| S021 | *36D | 9.85 | 2.1 | 3.2 | 5.6 | 8.3 |
| S017 | H120D, G195E, K235L | 9.40 | 2.9 | 5.4 | 10.8 | 14.1 |
| S025 | *36D,H120D,R170Y, K235L | 8.95 | 4.3 | 9.5 | 13.9 | 13.1 |
| | G195E,K235L | 8.05 | 9.6 | 13.0 | 12.4 | 9.2 |
| | G195E,K235L,K251E | 6.86 | 9.4 | 10.4 | 6.7 | 4.8 |

The results in Table X show clearly, that shifting the pI of a protease towards the pH of the wash liquor improves the wash performance of the protease.
The results also show, that all variants tested have improved performance compared to the wild type parent enzyme at pH below 10.0.

H:

The wash performance of various mutants was tested on grass juice stained cotton cloths according to the method described in Assay A.
2.0 g/l of liquid detergent D3 was used. The detergent was dissolved in 0,005 M glycine prepared in ion-exchanged water). pH was adjusted to 10.0, 10.25 , 10.50 , 10.75 , 11.0 , 11.5 , and 12.0, respectively, with NaOH. The temperature was kept at 30°C isothermic for 10 minutes.

The mutants were dosed at 0,2 mg enzyme protein/l each.

Results:

The wash performance of some of the enzyme variants of the invention at various pH values in a model system are shown in table XI. In this case variants with slightly higher pI than the wild type parent enzyme was investigated. The pH range from pH 10.0 to 12.0 is investigated in more details than in prior examples.

Table XI

| Variant | Mutation | | Delta R | | |
| --- | --- | --- | --- | --- | --- |
| | | $pI_0$ | pH 10.0 | 10.25 | 10.50 |
| S000 | | 10.06 | 7.0 | 8.7 | |
| 10.5 | | | | | |
| S027 | E89S | 10.28 | 6.0 | 8.5 | 9.8 |
| S028 | D181N | 10.28 | 6.9 | 9.8 | |
| 10.6 | | | | | |
| S032 | D197N | 10.28 | 4.7 | 9.2 | |
| 10.8 | | | | | |
| S033 | E271Q | 10.28 | 7.1 | 6.7 | 7.8 |
| S031 | D197N,E271Q | 10.53 | 4.7 | 7.2 | 7.0 |

| Variant | Mutation | | Delta R | | |
| --- | --- | --- | --- | --- | --- |
| | | $pI_0$ | pH 10.75 | 11.0 | 11.5 |
| S000 | | 10.06 | 12.5 | 14.4 | 10.6 | 3.8 |
| S027 | E89S | 10.28 | 11.9 | 14.3 | 12.8 | 5.0 |
| S028 | D181N | 10.28 | 13.0 | 14.4 | 10.7 | 4.6 |
| S032 | D197N | 10.28 | 13.8 | 13.5 | 11.3 | 5.0 |
| S033 | E271Q | 10.28 | 10.4 | 13.7 | 13.3 | 6.3 |
| S031 | D197N,E271Q | 10.53 | 10.7 | 13.0 | 14.4 | 8.7 |

The data in Table XI show, that at high pH values maximum performance is achieved at pH values a little above the calculated pI. Still increasing the pI of the protease tends to increase the pH of maximum performance. The effects are not as pronounced as it is seen at low pH values (assay B and G).

I:

In order to visualize the correlation between isoelectric point of the protease and the pH at which the protease has its maximum performance, the results from examples B, G, and H are used to find the pH at which each of the investigated variants (and the wild type parent enzyme) has its maximum performance. In Figure 5 this $pH_{max}$ is shown as a function of the calculated $pI_0$.

Taking into account, that the pH range is investigated in steps of 1.0 pH value the correlation is obvious.

Concerning the combination of the mutants of the invention with lipase experimental results led to the following practical conclusions:

Lipase was stable for an hour in wash liquor of type O at 37°C. The presence of Savinase® led to rapid deactivation. Kazusase® led to substantially less inactivation of lipase over the period of the test.

Proteinase K was seen to be less aggressive to lipase than Savinase®, but more so than Kazusase®. Subtilisin BPN' did not however inactivate lipase at all under these conditions.

Preferred proteases for use e.g. in connection with lipase in wash compositions represented by type 0, are mutants S001, S003, S004, S012, S019, S020, S021, S025, S035, and S235.

Type 0 wash liquor was a 5 g/l solution at 37°C derived from the following detergent formulation (% by wt):

| | |
| --- | --- |
| anionic surfactant | 6 |
| nonionic surfactant | 5 |
| fatty acid | 2.8 |
| acrylic polymer | 3 |

(continued)

| | |
|---|---|
| zeolite | 22 |
| carbonate | 10 |
| sulphate | 17.5 |
| clay | 8 |
| tertiary amine | 2 |
| perborate monohydrate | 13 |
| minors and water | to 100. |

Type W wash liquor was a 2 g/l solution of a liquid detergent having the following formulation (% by wt):

| | |
|---|---|
| anionic surfactant | 16 |
| nonionic surfactant | 7 |
| hydrotrope | 6 |
| citric acid | 6.5 |
| NaOH | 4.1 |
| monoethanolamine | 2 |
| minors and water | to 100. |

**Claims**

1. A method of preparing a mutated subtilisin protease for use in a detergent composition, which comprises changing the net electrostatic charge of the protease in comparison to the parent protease at the same pH, such that, in said protease there are, relative to said parent protease, fewer or more positively-charged amino acid residue(s) and/or more or fewer negatively-charged amino acid residue(s), wherein the parent protease is subtilisin 309 **characterized in that** a mutation is effected at a position corresponding to position 245 in subtilisin BPN' by substitution, whereby said mutated subtilisin protease has an isoelectric point ($pI_o$) lower or higher than that of said parent ptotease and the $pI_o$ of the mutated subtilisin protease is closer to the pH of the wash liquor formed by the detergent composition than is the $pI_o$ of the parent protease, such that the mutated subtilisin protease exhibits improved wash performance relative to the parent protease in said wash liquor.

2. The method of claim 1, **characterised in that** the protease possesses at least one further mutation affecting an amino acid residue occupying a position chosen from the group of positions
1, 2, 3, 4, 6, 9, 10, 12, 14, 15, 17, 18, 19, 20, 21, 22, 24, 25, 36, 37, 38, 40, 41, 43, 44, 45, 46, 49, 50, 51, 52, 53, 54, 55, 56, 57, 58, 59, 60, 61, 62, 75, 76, 77, 78, 79, 87, 89, 91, 94, 97, 98, 99, 100, 101, 103, 104, 105, 106, 107, 108, 109, 112, 113, 115, 116, 117, 118, 120, 126, 128, 129, 130, 131, 133, 134, 136, 137, 140, 141, 143, 144, 145, 146, 155, 156, 158, 159, 160, 161, 162, 163, 164, 165, 166, 167, 170, 171, 172, 173, 181, 182, 183, 184, 185, 186, 188, 189, 191, 192, 194, 195, 197, 204, 206, 209, 210, 211, 212, 213, 214, 215, 215, 217, 218, 235, 236, 237, 238, 239, 240, 241, 242, 243, 244, 247, 248, 249, 251, 253, 254, 255, 256, 257, 259, 260, 261, 262, 263, 265, 269, 271, 272, 275
whereby said mutated subtilisin protease has an isoelectric point ($pI_o$) lower or higher than that of said parent protease and the $pI_o$ of the mutated subtilisin protease is closer to the pH of the wash liquor formed by the detergent composition than is the $pI_o$ of the parent protease, such that the mutated subtilisin protease exhibits improved wash performance relative to the parent protease in said wash liquor.

**Patentansprüche**

1. Verfahren zum Herstellen einer mutierten Subtilisinprotease zur Verwendung in einer Detergenszusammensetzung, welches das Verändern der elektrostatischen Nettoladung der Protease im Vergleich zu der Ausgangsprotease bei dem gleichen pH umfasst, so dass in der Protease bezüglich der Ausgangsprotease weniger oder mehr positiv geladene Aminosäurerest(e) und/oder mehr oder weniger negativ geladene Aminosäurerest(e) sind, wobei die Ausgangsprotease Subtilisin 309 ist, **dadurch** charakterisiert, dass eine Mutation durch Substitution an einer Position bewirkt ist, die Position 245 in Subtilisin BPN' entspricht, wobei die mutierte Subtilisin-Protease einen isoelektrischen

Punkt (pI$_o$) hat, der niedriger oder höher als der der Ausgangsprotease ist, und der pI$_o$ der mutierten Subtilisinprotease näher zu dem pH der Waschflotte ist, die durch die Detergenszusammensetzung gebildet wird, als der pI$_o$ der Ausgangsprotease, so dass die mutierte Subtilisinprotease eine verbesserte Waschleistung bezüglich der Ausgangsprotease in der Waschflotte aufweist.

**2.** Verfahren nach Anspruch 1, **dadurch gekennzeichnet, dass** die Protease mindestens eine weitere Mutation besitzt, betreffend einen Aminosäurerest, der eine Position besetzt, ausgewählt aus der Gruppe von Positionen 1, 2, 3, 4, 6, 9, 10, 12, 14, 15, 17, 18, 19, 20, 21, 22, 24, 25, 36, 37, 38, 40, 41, 43, 44, 45, 46, 49, 50, 51, 52, 53, 54, 55, 56, 57, 58, 59, 60, 61, 62, 75, 76, 77, 78, 79, 87, 89, 91, 94, 97, 98, 99, 100, 101, 103, 104, 105, 106, 107, 108, 109, 112, 113, 115, 116, 117, 118, 120, 126, 128, 129, 130, 131, 133, 134, 136, 137, 140, 141, 143, 144, 145, 146, 155, 156, 158, 159, 160, 161, 162, 163, 164, 165, 166, 167, 170, 171, 172, 173, 181, 182, 183, 184, 185, 186, 188, 189, 191, 192, 194, 195, 197, 204, 206, 209, 210, 211, 212, 213, 214, 215, 216, 217, 218, 235, 236, 237, 238, 239, 240, 241, 242, 243, 244, 247, 248, 249, 251, 253, 254, 255, 256, 257, 259, 260, 261, 262, 263, 265, 269, 271, 272, 275, wobei die mutierte Subtilisinprotease einen isoelektrischen Punkt (pI$_o$) hat, der niedriger oder höher ist als der der Ausgangsprotease, und der pI$_o$ der mutierten Subtilisinprotease näher zu dem pH der Waschflotte ist, der durch die Detergenszusammensetzung gebildet wird, als der pI$_o$ der Ausgangsprotease, so dass die mutierte Subtilisinprotease eine verbesserte Waschleistung bezüglich der Ausgangsprotease in der Waschflotte aufweist.

## Revendications

**1.** Procédé pour préparer une protéase subtilisine mutée pour une utilisation dans une composition détergente, qui consiste à changer la charge électrostatique nette de la protéase par comparaison avec la protéase parente au même pH, de telle sorte que, dans ladite protéase, il y ait, par rapport à ladite protéase parente, moins ou plus de résidus d'acides aminés chargés positivement et/ou plus ou moins de résidus d'acides aminés chargés négativement, dans lequel la protéase parente est la subtilisine 309, **caractérisée en ce qu'**une mutation est effectuée en une position correspondant à la position 245 dans la subtilisine BPN' par substitution, grâce à quoi ladite protéase subtilisine mutée a un point isoélectrique (pI$_o$) inférieur ou supérieur à celui de ladite protéase parente et le pI$_o$ de la protéase subtilisine mutée est plus proche du pH de la liqueur de lavage formée par la composition détergente que ne l'est le pI$_o$ de la protéase parente, si bien que la protéase subtilisine mutée présente une meilleure performance de lavage que la protéase parente dans ladite liqueur de lavage.

**2.** Procédé selon la revendication 1, **caractérisé en ce que** la protéase possède au moins une autre mutation affectant un résidu d'acide aminé occupant une position choisie dans le groupe des positions 1, 2, 3, 4, 6, 9, 10, 12, 14, 15, 17, 18, 19, 20, 21, 22, 24, 25, 36, 37, 38, 40, 41, 43, 44, 45, 46, 49, 50, 51, 52, 53, 54, 55, 56, 57, 58, 59, 60, 61, 62, 75, 76, 77, 78, 79, 87, 89, 91, 94, 97, 98, 99, 100, 101, 103, 104, 105, 106, 107, 108, 109, 112, 113, 115, 116, 117, 118, 120, 126, 128, 129, 130, 131, 133, 134, 136, 137, 140, 141, 143, 144, 145, 146, 155, 156, 158, 159, 160, 161, 162, 163, 164, 165, 166, 167, 170, 171, 172, 173, 181, 182, 183, 184, 185, 186, 188, 189, 191, 192, 194, 195, 197, 204, 206, 209, 210, 211, 212, 213, 214, 215, 216, 217, 218, 235, 236, 237, 238, 239, 240, 241, 242, 243, 244, 247, 248, 249, 251, 253, 254, 255, 256, 257, 259, 260, 261, 262, 263, 265, 269, 271, 272, 275, grâce à quoi ladite protéase subtilisine mutée a un point isoélectrique (pI$_o$) inférieur ou supérieur à celui de ladite protéase parente et le pI$_o$ de la protéase subtilisine mutée est plus proche du pH de la liqueur de lavage formée par la composition détergente que ne l'est le pI$_o$ de la protéase parente, si bien que la protéase subtilisine mutée présente une meilleure performance de lavage que la protéase parente dans ladite liqueur de lavage.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 130756 A
- US 4760025 A
- EP 214435 A
- WO 8704461 A
- WO 8705050 A
- EP 87303761 A
- EP 260105 A
- WO 8806624 A
- WO 8807578 A
- WO 8808028 A
- WO 8808033 A
- WO 8808164 A
- DK 8800002 W
- EP 0283075 A
- US 8801040 W

- EP 0205208 A
- EP 0206390 A
- EP 258068 A
- EP 214761 A
- EP 0258068 A
- WO 8700859 A
- EP 0204284 A
- WO 8802775 A
- EP 0243338 A
- EP 0268452 A
- EP 0305216 A
- US 3723250 A
- US 039298 A
- EP 242220 A
- EP 0342177 A

**Non-patent literature cited in the description**

- **Walsh.** Enzymatic Reaction Mechanisms. W.H. Freeman and Company, 1979
- **Joliffe et al.** *J. Bacterial,* 1980, vol. 141, 1199-1208
- **White ; Handler ; Smith.** Principles of Biochemistry. McGraw-Hill Book Company, 1973, 271-272
- **Priest.** *Bacteriological Rev.,* 1977, vol. 41, 711-753
- **Kurihara et al.** *J.Biol.Chem.,* 1972, vol. 247, 5629-5631
- **Wells et al.** *Nucleic Acids Res.,* 1983, vol. 11, 7911-7925
- **Stahl ; Ferrari.** *J.Bacteriol.,* 1984, vol. 159, 811-819
- **Jacobs et al.** *Nucl.Acids Res.,* 1985, vol. 13, 8913-8926
- **Nedkov et al.** *Biol.Chem. Hoppe-Seyler,* 1985, vol. 366, 421-430
- **Svendsen et al.** *FEBS Lett,* 1986, vol. 196, 228-232
- **Meloun et al.** *FEBS Lett.,* 1985, vol. 1983, 195-200
- **Jany ; Mayer.** *Biol.Chem. Hoppe-Seyler,* 1985, vol. 366, 584-492
- **Kraut.** *Ann.Rev.Biochem.,* 1977, vol. 46, 331-358
- **Wells et al.** *Proc.Natl.Acad.Sci. U.S.A.,* 1987, vol. 84, 1219-1223

- **Wells et al.** *Phil.Trans.R.Soc.Lond.A.,* 1986, vol. 317, 415-423
- **Hwang ; Warshel.** *Biochem.,* 1987, vol. 26, 2669-2673
- **Rao et al.** *Nature,* 1987, vol. 328, 551-554
- **Thomas ; Russell ; Fersht.** *Nature,* 1985, vol. 318, 375-376
- *J. Mol. Biol.,* 1987, vol. 193, 803-813
- **Russel ; Fersht.** *Nature,* 1987, vol. 328, 496-500
- **Betzel et al.** *Eur. J. Biochem.,* 1988, vol. 178, 155 ff
- **Gunkel et al.** *Eur. J. Biochem.,* 1989, vol. 179, 185 ff
- **Kwon et al.** *Eur. J. Biochem.,* 1988, vol. 173, 491 ff
- **Shortle ; Nathans.** *Proc. Natl. Acad. Sci. U.S.A.,* 1978, vol. 75, 2170-2174
- **Myers et al.** *Science,* 1985, vol. 229, 242-257
- **Morinaga et al.** *Biotechnology,* 1984, vol. 2, 646-639
- **Villa-Kamaroff et al.** *Proc. Natl. Acad. Sci. U.S.A.,* 1978, vol. 75, 3727-3731
- **DeBoer et al.** *Proc. Natl. Acad. Sci. U.S.A.,* 1983, vol. 80, 21-25
- Useful proteins from recombinant bacteria. *Scientific American,* 1980, vol. 242, 74-94
- **Casadaban, M.J. ; Cohen, S.N.** *J. Mol. Biol.,* 1980, vol. 138, 179-207

- *J.Bacteriol.,* vol. 160 (2), 442-444
- **Vieira ; Messing.** *Gene,* 1982, vol. 19, 259-268
- **Diderichsen, B. ; Christiansen, L.** *FEMS Microbiology Letters,* 1988, vol. 56, 53-60
- **Takkinen et al.** *J. Biol. Chem.,* 1983, vol. 258, 1007ff
- **Vieira, J. ; Messing, J.** *Gene,* 1982, vol. 19, 259-268
- **Yanisch-Perron, C. ; Vieira, J. ; Messing, J.** *Gene,* 1985, vol. 33, 103-119
- **Lacey,R.W. ; Chopra,J.** Genetic studies of a multiresistant strain of Staphylococcus aureus. *J. Med.Microbiol.,* 1974, vol. 7, 285-297
- **Zyprian, E. ; Matzura,H.** Characterization of signals promoting gene expression on the Staphylococcus aureus plasmid pUB110 and development of a Gram-positive expression vector system. *DNA,* 1986, vol. 5 (3), 219-225
- **Jacobs, M. ; Eliasson,M. ; Uhlen,M. ; Flock,J.-I.** Cloning, sequencing and expression of subtilisin Carlsberg from Bacillus licheniformis. *Nucleic Acids Res.,* 1985, vol. 13 (24), 8913-8926
- **Wells,J.A. ; Ferrari,E. ; Henner,D.J. ; Estell,D.A. ; Chen,E.Y.** Cloning, sequencing and secretion of Bacillus amyloliquefaciens subtilisin in Bacillus subtilis. *Nuclei Acids Res.,* 1983, vol. 11 (22), 7911-7925
- **Mullenbach, G.T. ; Tabrizi, A. ; Blacher, R.W. ; Steimer,K.S.** Chemical synthesis and expression in Yeast of a gene encoding connective tissue activating peptide-III. *J.Biol. Chem.,* 1986, vol. 261 (2), 719-722
- **Kawamura,F. ; Doi, R.H.** Construction of a Bacillus subtilis double mutant deficient in extracellular alkaline and neutral proteases. *J.Bacteriol.,* 1984, vol. 160 (2), 442-444
- **Zyprian,E. ; Matzura,H.** Characterization of signals promoting gene expression on the Staphylococus aureus plasmid pUB110 and development of a Gram-positive expression vector system. *DNA,* 1986, vol. 5 (3), 219-225
- **Smith,E.L. ; DeLange,R.J. ; Evans,W.H. ; Landon,W. ; Markland,F.S. ; Subtilisin Carlsberg V.** The complete sequence: comparison with subtilisin BPN'; evolutionary relationships. *J.Biol.-Chem.,* 1968, vol. 243 (9), 2184-2191
- **Takkinen et al.** *J. Biol. Chem.,* 1983, vol. 258, 1007ff